(19) Europäisches Patentamt
European Patent Office
Office européen des brevets

(11) EP 2 253 621 A1

(12) EUROPEAN PATENT APPLICATION
published in accordance with Art. 153(4) EPC

(43) Date of publication:
24.11.2010 Bulletin 2010/47

(21) Application number: 09703551.3

(22) Date of filing: 19.01.2009

(51) Int Cl.:
C07D 225/06 (2006.01)
C07H 19/06 (2006.01)
C07D 401/12 (2006.01)
A61K 31/395 (2006.01)
A61K 31/4427 (2006.01)
A61P 31/12 (2006.01)
A61P 35/00 (2006.01)

(86) International application number:
PCT/CN2009/000074

(87) International publication number:
WO 2009/092295 (30.07.2009 Gazette 2009/31)

(84) Designated Contracting States:
AT BE BG CH CY CZ DE DK EE ES FI FR GB GR HR HU IE IS IT LI LT LU LV MC MK MT NL NO PL PT RO SE SI SK TR
Designated Extension States:
AL BA RS

(30) Priority: 18.01.2008 CN 200810001170

(71) Applicant: Institute Of Medicinal Biotechnology, Chinese
Academy of Medical Sciences
Chongwen
100050 Beijing (CN)

(72) Inventors:
- LI, Zhuorong
Beijing 100050 (CN)
- PENG, Zonggen
Beijing 100050 (CN)
- LI, Yanping
Beijing 100050 (CN)
- ZHU, Jianhua
Beijing 100050 (CN)
- TAO, Peizhen
Beijing 100050 (CN)
- FAN, Bo
Beijing 100050 (CN)
- WANG, Yuping
Beijing 100050 (CN)
- SHAN, Guangzhi
Beijing 100050 (CN)
- WANG, Shuqin
Beijing 100050 (CN)
- ZHANG, Tian
Beijing 100050 (CN)
- JIANG, Jiandong
Beijing 100050 (CN)

(74) Representative: Samson & Partner
Widenmayerstraße 5
80538 München (DE)

# (54) A SET OF GELDANAMYCIN DERIVATIVES AND THEIR PREPARATION METHODS

(57) A set of geldanamycin derivatives and their preparation methods. Pharmaceutical compositions comprising the said compounds as an active ingredient which are used as antivirus and antitumor agents. The said derivatives are used in the manufacture of heat shock protein 90(Hsp 90) inhibiting agents which have the utility as antivirus and antitumor agents.

EP 2 253 621 A1

## Description

### Technical field

The invention relates to a series of structurally modified derivatives of geldanamycin, the preparation methods of the said compounds, their applications in anti-virus and anti-tumor, and pharmaceutical composition of the said compounds.

### Background

Geldanamycin is a benzoquinone ansamycin antibiotic generated by fermentation of Streptomyces hygroscopicus. Its molecule composes of a benzoquinone structure and a planar macrocyclic ansamycin bridge. The target of geldanamycin is heat shock protein 90(Hsp90), it deactivates Hsp90 specifically to inhibit tumor growth or virus replication. Through interfering normal functioning of Hsp90, geldanamycin holds back the activation of the substrate protein of Hsp90, induces interdiction of cell cycle and inhibits virus replication, thereby exerting anti-virus and anti-tumor effects. The unique mechanism of geldanamycin makes itself with broad anti-virus and anti-tumor spectra, it suffers no cross resistance of the subjects with other medicines and its subjects are difficult to generate resistance against it. Geldanamycin is an excellent lead compound for new anti-virus and anti-tumor drugs using cytokine as their targets.

With the study of Hsp90 inhibitor screening as the main object, the Institute of Medicinal Biotechnology, Chinese Academy of Medical Sciences carried out a series of studies on geldanamycin, it possesses a patent on the usage of geldanamycin as a anti-virus infection drug (ZL97100523), studied in depth the anti-virus activity and mechanism of geldanamycin, as well as its application development (Li Yuhuan, Tao Pei-Heng et al: Antimicrobial Agents and Chemotherapy, 48(3): 867-872; 2004). On the basis of synthesis and study on the anti-virus effect of geldanamycin 17-Nucleoside derivatives (CN1817866A), the applicant of this invention has further synthesized a series of new 17- modified as well as 17- and 19- simultaneously modified derivatives of geldanamycin, and has tested the anti-virus activities of the compounds. Up to now, no published reports on said modified derivatives of geldanamycin and their anti-virus activities have been seen in the literature in China as well as abroad.

A main object of this invention is to obtain new types of Hsp90 inhibitor with weaker toxicities through introduction of various substitutes in 17- and/or 19-positions of geldanamycin molecules, while they retain or strengthen the original anti-virus activity of geldanamycin. The achievements studied out these new types of Hsp90 inhibitor with weaker toxicities and higher efficiencies can lay a foundation for further studies and developments on anti-virus and anti-tumor medicines with Hsp90 as target.

### Summary of the invention

This invention provides a series of structurally modified derivatives of geldanamycin, whose structures are shown in Formula (I):

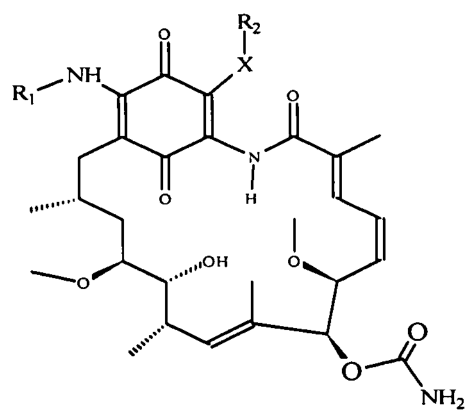

(I)

wherein:

$R_1$ is a substituent which has a linkage moiety on its one end consisting of linear or branched, saturated or unsaturated chain containing 3 to 20 carbon atoms and containing or not containing ether, ester or amide bonds in said chain, and the other end of the substituent is an alicyclic or aromatic cyclic group which may optionally be substituted by hydrocarbyl, halogen, hydroxyl, carboxyl, nitrile group, amino, sulfonic or phosphoric acid group or esters or salts thereof;
$R_2$ is H or a same substituent as $R_1$ or a different substituent from $R_1$;
X is NH, O or S; or X-$R_2$ is H.

The preparation of Formula (I) compounds can be realized by using the following general method. The amine containing substituent $R_1$ is synthesized or purchased, and is allowed to react with geldanamycin in a haloalkane, alcoholic or polar aprotic solvent (N,N-dimethylformamide, dimethylsulfoxide, ethyl acetate, acetonitrile, or acetone) and under alkaline condition (triethylamine, pyridine, N, N-dimethyl pyridine, potassium carbonate, sodium carbonate, or calcium hydroxide) to obtain 17-mono substituted compound (I, X-$R_2$ is H). The 17-, 19-bisubstituted compounds are prepared by reacting 17-monosubstituted compounds used as material with $R_2$XH under the similar condition to obtain target 17-, 19- bisubstituted compounds (I, both $R_1$ and X-$R_2$ are not H).

When the other side of $R_1$ is 3, 4-di-hydroxyl- methylated caffeic acid moiety, the preparation of Formula (I) compounds can be realized according to the following route: firstly, caffeic acid reacts with a methyalting reagent (dimethyl sulfate, methyl methanesulfonate, methyl iodide, dimethyl carbonate) under alkaline condition to obtain 3,4-di-hydroxyl-methylated caffeic acid. The latter is reacted with acyl chlorinating reagent to obtain the acyl chloride, which reacts subsequently with mono N-tert-butoxycarbonyl-ethylenediamine

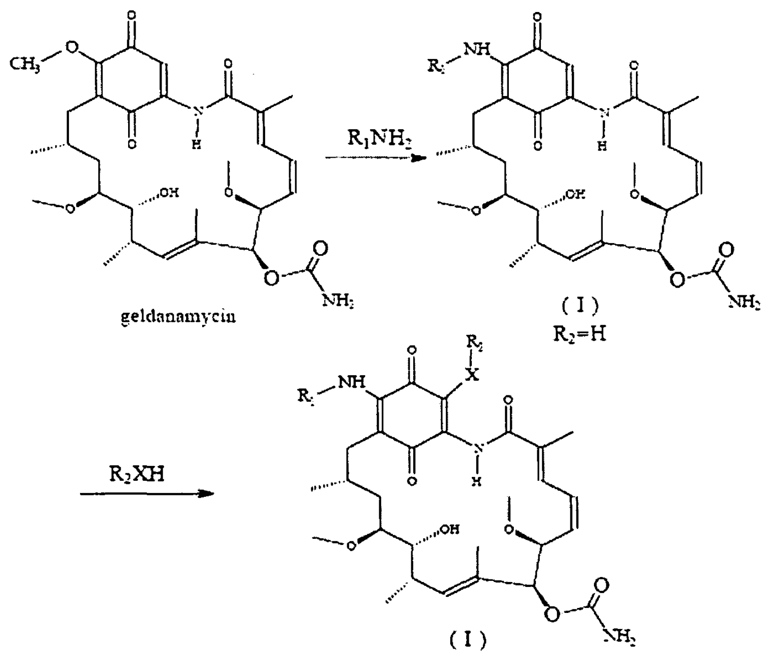

geldanamycin
( I )
R2=H
( I )

to obtain (2-tert-butoxycarbonylamino) ethyl-3,4-di-hydroxyl-methylated caffeoyl amide. After removal of tert-Butyl protective group, (2-amino)ethyl-3,4 -di-hydroxyl-methylated caffeoylamide is obtained. The latter is subsequently reacted with geldanamycin using the method similar to the aforementioned to produce geldanamycin derivative containing di-hydroxyl-methylated caffeoylamide moiety in the 17- position of the compound.

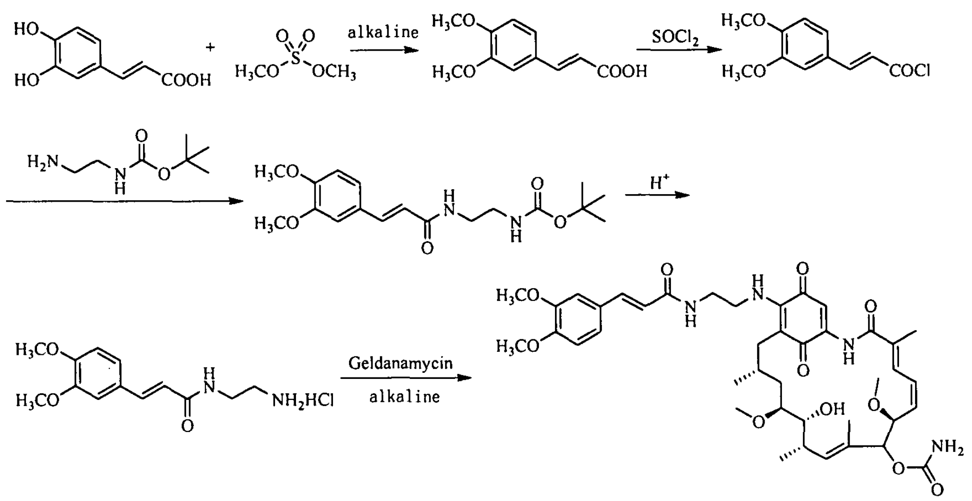


When the other side of $R_1$ is a cytidine moiety, the preparation of the compounds of Formula (I) structure is as follows: cytidine is reacted with 2, 2-dimethoxypropane under acidic condition to obtain 2', 3'-isopropylidene cytidine. Under the effect of a dehydrating reagent (DCC, TBU), the product condensates with γ-*tert*-Butoxycarbonylamino butyric acid to obtain esterification product of the acid with 2', 3'- isopropylidene cytidine. After removal of the BOC protective group by alcoholysis under acidic catalysis, cytidine γ-aminobutyrate hydrochloride is obtained. Finally, geldanamycin derivative with 17-cytidine moiety is produced by reacting cytidine γ-aminobutyrate hydrochloride with geldanamycin using the method similar to the aforementioned.

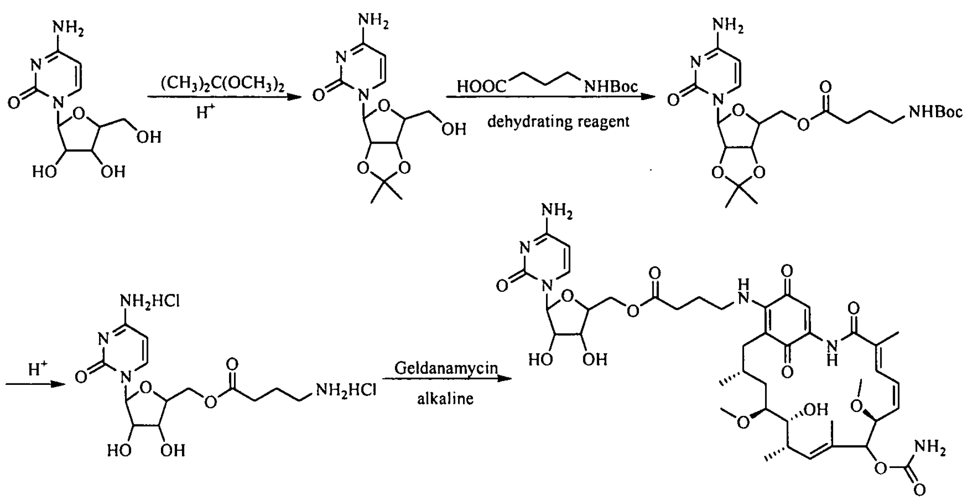


When the other side of $R_1$ is a niacinamido moiety, the preparation of the compound of Formula (I) structure is as follows: the nicotinoyl chloride produced by reacting nicotinic acid with acyl chlorination reagent (dichlorosulfoxide) is reacted with 2-(N-*tert*-butyloxycarbonyl) ethanediamine to obtain 2-(tert-butoxycarbonylamino) ethyl niacinamide. After removal of the protective group under acidic catalysis, (2-amino) ethyl niacinamide is obtained, which is finally reacted with geldanamycin to produce geldanamycin derivatives with 17-niacinamido moiety using the method similar to the aforementioned.

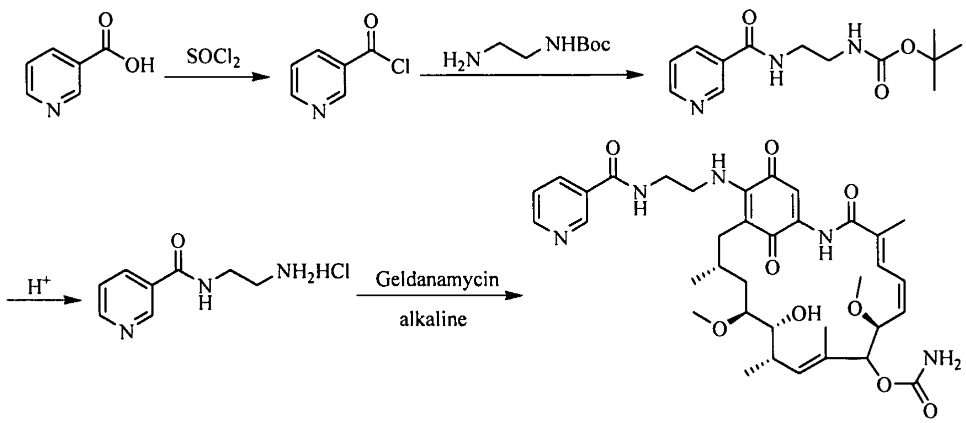


When the other side of $R_1$ is a phosphonate moiety, the preparation of the compound of Formula (I) structure is as follows: Phthalimide potassium salt is reacted with *p*-tolyl sulfonyloxoalkyl phosphonate diethyl ester in a polar aprotic solvent, to produce *N*-alkylphosphonate diethyl ester-phthalimide, which further reacts with hydrazine hydrate to produce aminoalkyl phosphonate diethyl ester.

Finally, geldanamycin derivative with 17-phosphonate moiety is produced by reacting aminoalkyl phosphonate diethyl ester with geldanamycin using the method similar to the aforementioned.

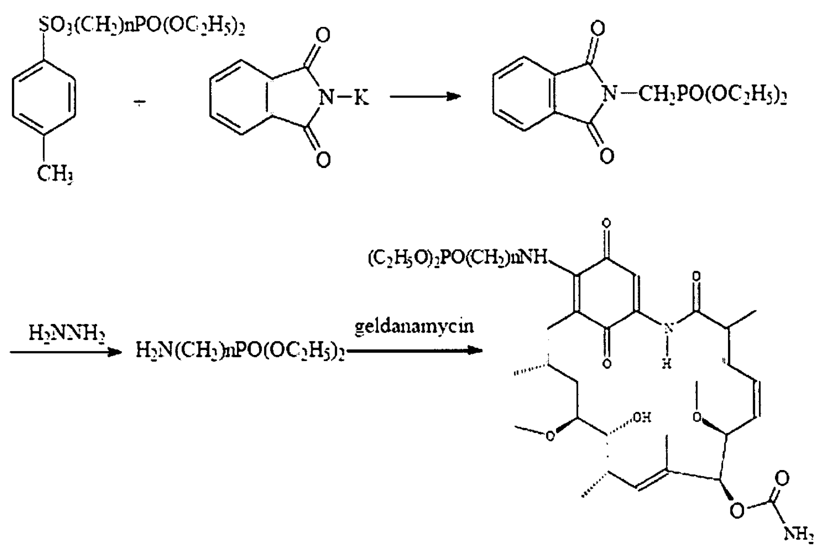


All compounds comprised in this invention can be prepared according to aforementioned reaction route and method (Table 1).

Compounds having the structure of Formula (I) are tested for their anti-HBV, anti-HIV and anti-HSV activities. Based upon the mechanism of geldanamycin effect on Hsp90, geldanamycin has simultaneously anti-tumor activity.

This invention also provides the pharmaceutical compositions containing said compounds with therapeutically effective amount as the active components and one or more pharmacologically acceptable carriers.

The compounds and compositions provided by this invention can be used to prepare anti-virus and anti-tumor medicines.

Various formulations of the medicinal compositions provided by this invention can be prepared according to the conventional production methods in the realm of pharmacy, for example, mixing of an active ingredient with one or more kinds of carriers, and subsequently prepare the formulations needed.

The medicinal compositions prepared herein are preferably those containing 0.1%-99.5% weight ratio of the active ingredients, the most preferably weight ratio of the active ingredients are in the range of 0.5%-99.5%.

The effect of this invention

According to the aforementioned reaction routes and methods, a series of new derivatives of geldanamycin described herein can be obtained steadily and reproducibly. The result of tests on the biological activities and pharmacologies showed that the said derivatives has broad-spectrum anti-virus activities, especially showed relatively stronger inhibition effect against HIV-1and HBV viruses. What is more, the compounds described herein also showed relatively strong inhibition activities against HSV. The structures of the said compounds and their activities measured are shown in Table 1.

Table 1 Structures and Anti-virus Activities of the Compounds in This Invention

| No. of compound | characteristics | MW | Molecular formula | $R_1$ | $R_2$ | Activity of HIV inhibition $IC_{50}$,μg/ml | Activity of HBV inhibition $IC_{50}$,μg/ml |
|---|---|---|---|---|---|---|---|
| GM-APML | purple solid | 658.78 | $C_{34}H_{50}N_4O_9$ | 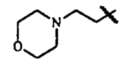 | H | <0.01 | 0.064 |
| GM-AEPD | purple solid | 656.81 | $C_{35}H_{52}N_4O_8$ | 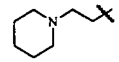 | H | <0.01 | 0.08 |
| GM-ABPD | purple solid | 718.88 | $C_{40}H_{54}N_4O_8$ | 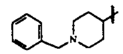 | H | <0.03 | 0.32 |
| GM-AMPP | purple solid | 642.78 | $C_{34}H_{50}N_4O_8$ | 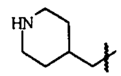 | H | 0.06 | 8.0 |
| GM-MTA | purple solid | 681.86 | $C_{38}H_{55}N_3O_8$ | 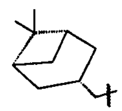 | H | 0.04 | 0.32 |
| GM-GP | purple solid | 695.74 | $C_{33}H_{50}N_3O_{11}P$ | 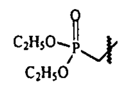 | H | 1.36 | 3.24 |
| GM-129 | purple solid | 653.77 | $C_{34}H_{47}N_5O_8$ | 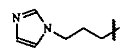 | H | 1.92 | 0.32 |
| GM-208 | purple solid | 728.85 | $C_{36}H_{48}N_4O_{10}S$ | 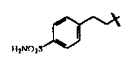 | H | >0.82 | |
| GM-217 | purple solid | 679.76 | $C_{36}H_{45}N_3O_{10}$ | 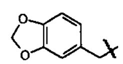 | H | 0.16 | |
| GM-221 | purple solid | 655.8 | $C_{34}H_{45}N_3O_8S$ | 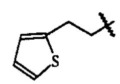 | H | 0.06 | |

(continued)

| No. of compound | characteristics | MW | Molecular formula | $R_1$ | $R_2$ | Activity of HIV inhibition $IC_{50}$,μg/ml | Activity of HBV inhibition $IC_{50}$,μg/ml |
|---|---|---|---|---|---|---|---|
| GM-223 | purple solid | 643.77 | $C_{34}H_{49}N_3O_9$ | 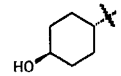 | H | 0.10 | |
| GM-226 | purple solid | 670.79 | $C_{35}H_{50}N_4O_9$ | 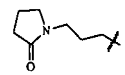 | H | >0.14 | 1.6 |
| GM-228 | purple solid | 656.81 | $C_{35}H_{52}N_4O_8$ | 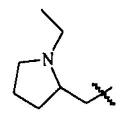 | H | 0.009 | 0/01 |
| GM-206S | purple solid | 656.81 | $C_{35}H_{52}N_4O_8$ | 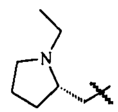 | H | 0.01 | 0.064 |
| GM-210R | purple solid | 656.81 | $C_{35}H_{52}N_4O_8$ | 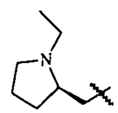 | H | 0.01 | 0.064 |
| GM-413 | purple solid | 778.89 | $C_{41}H_{54}N_4O_{11}$ | 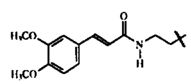 | H | 0.48 | 0.32 |
| GM-418 | purple solid | 693.79 | $C_{36}H_{47}N_5O_9$ | 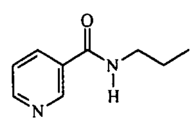 | H | 0.37 | 0.08 |
| GM-CY | purple solid | 856.92 | $C_{41}H_{56}N_6O_{14}$ | 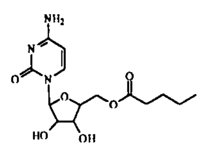 | H | 0.02 | 0..032 |

(continued)

| No. of compound | characteristics | MW | Molecular formula | $R_1$ | $R_2$ | Activity of HIV inhibition $IC_{50}$, μg/ml | Activity of HBV inhibition $IC_{50}$, μg/ml |
|---|---|---|---|---|---|---|---|
| THFM(R)-GM | purple solid | 629.74 | $C_{33}H_{47}N_3O_9$ | 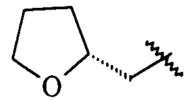 | H | 0.05 | 0.01 |
| THFM(S)-GM | purple solid | 629.74 | $C_{33}H_{47}N_3O_9$ | 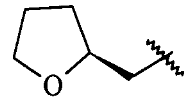 | H | 0.06 | 0.032 |
| THFM-GM | purple solid | 629.74 | $C_{33}H_{47}N_3O_9$ | 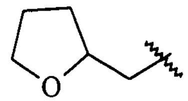 | H | 0.14 | 0.01 |
| THFM-GM2 | purple solid | 728.87 | $C_{38}H_{56}N_4O_{10}$ | 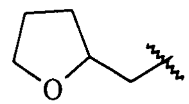 | 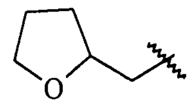 | 0.24 | 0.064 |
| THFM-11 | purple solid | 728.87 | $C_{38}H_{56}N_4O_{10}$ | 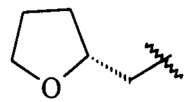 | 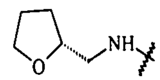 | >22.2 | 0.013 |
| THFM+2 | purple solid | 728.87 | $C_{38}H_{56}N_4O_{10}$ | 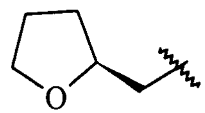 | 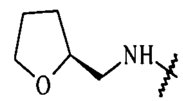 | 0.18 | 0.10 |
| GM-W1 | purple solid | 636.74 | $C_{34}H_{44}N_4O_8$ | 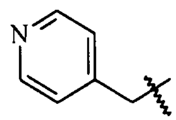 | H | 36.7 | 0.69 |
| GM-W2 | purple solid | 651.75 | $C_{34}H_{45}N_5O_8$ | 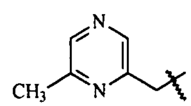 | H | 19.6 | 8.0 |

(continued)

| No. of compound | characteristics | MW | Molecular formula | $R_1$ | $R_2$ | Activity of HIV inhibition $IC_{50}$,μg/ml | Activity of HBV inhibition $IC_{50}$,μg/ml |
|---|---|---|---|---|---|---|---|
| GM-W3 | purple solid | 667.68 | $C_{31}H_{46}N_3O_{11}P$ | 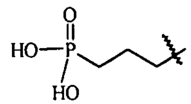 | H | 1.89 | 1.32 |
| GM-W4 | purple solid | 654.71 | $C_{33}H_{42}N_4O_{10}$ | 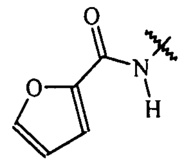 | H | 0.33 | 3.2 |
| GM-W5 | purple solid | 653.74 | $C_{30}H_{43}N_3O_{11}S$ | 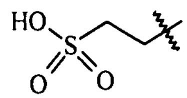 | H | 10.26 | 6.4 |
| GM-W6 | purple solid | 633.73 | $C_{32}H_{47}N_3O_{10}$ | 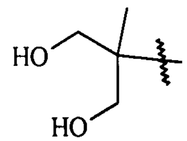 | H | 1.47 | 1.32 |
| GM-W7 | purple solid | 617.73 | $C_{32}H_{47}N_3O_9$ | 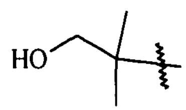 | H | 9.89 | 8.0 |
| GM-W8 | purple solid | 665.77 | $C_{36}H_{47}N_3O_9$ | 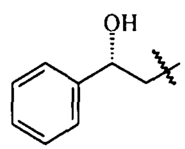 | H | 1.22 | 1.32 |
| GM-W9 | purple solid | 665.77 | $C_{36}H_{47}N_3O_9$ | 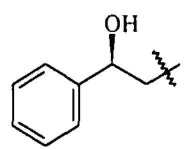 | H | 2.46 | 3.2 |

(continued)

| No. of compound | characteristics | MW | Molecular formula | $R_1$ | $R_2$ | Activity of HIV inhibition $IC_{50}$,μg/ml | Activity of HBV inhibition $IC_{50}$,μg/ml |
|---|---|---|---|---|---|---|---|
| GM-W10 | purple solid | 664.79 | $C_{36}H_{48}N_4O_8$ | 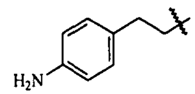 | H | 40.71 | 6.4 |
| GM-W11 | purple solid | 628.76 | $C_{33}H_{48}N_4O_8$ | 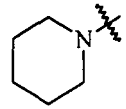 | H | 3.87 | 8.0 |
| GM-W12 | purple solid | 633.73 | $C_{32}H_{47}N_3O_{10}$ | 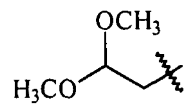 | H | 0.46 | 3.2 |
| GM-W13 | purple solid | 584.66 | $C_{30}H_{40}N_4O_8$ | 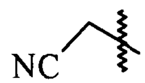 | H | 0.65 | 0.64 |
| GM-W14 | purple solid | 617.73 | $C_{32}H_{47}N_3O_9$ | 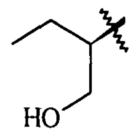 | H | 3.91 | 0.32 |
| GM-W15 | purple solid | 617.73 | $C_{32}H_{47}N_3O_9$ | 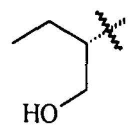 | H | 1.47 | 0.01 |
| GM-W16 | purple solid | 631.76 | $C_{33}H_{49}N_3O_9$ | 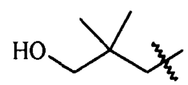 | H | 0.43 | 0.064 |
| GM-W17 | purple solid | 669.74 | $C_{30}H_{43}N_3O_{12}S$ | 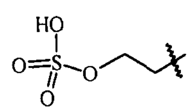 | H | 23.47 | 10.3 |

(continued)

| No. of compound | characteristics | MW | Molecular formula | $R_1$ | $R_2$ | Activity of HIV inhibition $IC_{50}$,μg/ml | Activity of HBV inhibition $IC_{50}$,μg/ml |
|---|---|---|---|---|---|---|---|
| GM-W18 | purple solid | 650.76 | $C_{35}H_{46}N_4O_8$ | 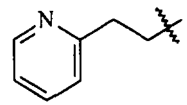 | H | 33.41 | 50.4 |
| GM-W19 | purple solid | 636.74 | $C_{34}H_{44}N_4O_8$ | 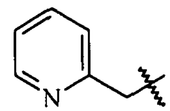 | H | 28.90 | 3.4 |
| GM-W20 | purple solid | 636.74 | $C_{34}H_{44}N_4O_8$ | 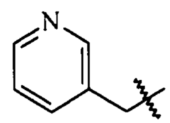 | H | 26.42 | 6.89 |
| GM-W21 | purple solid | 661.78 | $C_{37}H_{47}N_3O_8$ | 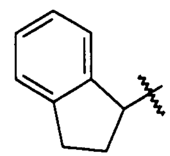 | H | 58.97 | 10.4 |
| GM-W22 | purple solid | 642.78 | $C_{34}H_{50}N_4O_8$ | 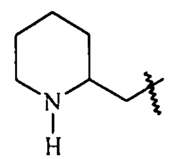 | H | 1.37 | 3.2 |
| GM-W23 | purple solid | 642.78 | $C_{34}H_{50}N_4O_8$ | 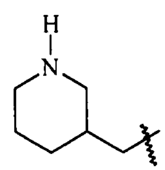 | H | 0.46 | 0.32 |

(continued)

| No. of compound | characteristics | MW | Molecular formula | $R_1$ | $R_2$ | Activity of HIV inhibition $IC_{50}$,μg/ml | Activity of HBV inhibition $IC_{50}$,μg/ml |
|---|---|---|---|---|---|---|---|
| GM-W24 | purple solid | 601.69 | $C_{30}H_{43}N_5O_8$ | 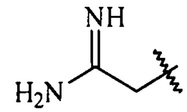 | H | 0.11 | 0.01 |
| GM-W25 | purple solid | 633.73 | $C_{32}H_{47}N_3O_{10}$ | 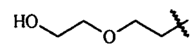 | H | 0.43 | 0.32 |
| GM-W26 | purple solid | 632.74 | $C_{32}H_{48}N_4O_9$ | 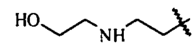 | H | 0.21 | 0.64 |
| GM-W27 | purple solid | 746.93 | $C_{42}H_{58}N_4O_8$ | 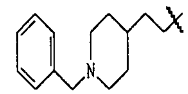 | H | 13.81 | 3.9 |
| GM-W28 | purple solid | 645.74 | $C_{33}H_{47}N_3O_{10}$ | 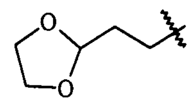 | H | 0.21 | 0.10 |
| GM-W29 | purple solid | 631.71 | $C_{32}H_{45}N_3O_{10}$ | 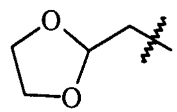 | H | 0.67 | 6.4 |
| GM-W30 | purple solid | 706.87 | $C_{39}H_{54}N_4O_8$ | 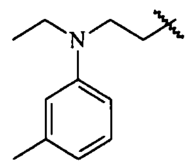 | H | 10.89 | 5.6 |
| GM-W31 | purple solid | 631.76 | $C_{33}H_{49}N_3O_9$ | 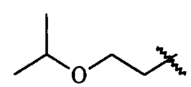 | H | 0.32 | 0.32 |

(continued)

| No. of compound | characteristics | MW | Molecular formula | $R_1$ | $R_2$ | Activity of HIV inhibition $IC_{50}$,μg/ml | Activity of HBV inhibition $IC_{50}$,μg/ml |
|---|---|---|---|---|---|---|---|
| GM-W32 | purple solid | 650.76 | $C_{35}H_{46}N_4O_8$ | 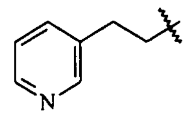 | H | 0.48 | 6.4 |
| GM-W33 | purple solid | 658.78 | $C_{34}H_{50}N_4O_9$ | 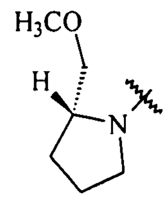 | H | 0.23 | 3.2 |
| GM-W34 | purple solid | 658.78 | $C_{34}H_{50}N_4O_9$ | 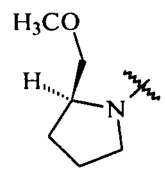 | H | 0.57 | 0.17 |
| GM-W35 | purple solid | 643.77 | $C_{33}H_{49}N_5O_8$ | 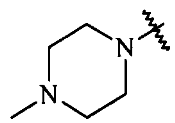 | H | 3.48 | 4.36 |
| GM-W36 | purple solid | 665.77 | $C_{36}H_{47}N_3O_9$ | 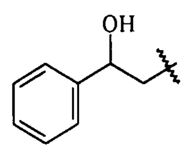 | H | 0.29 | 0.02 |
| GM-W37 | purple solid | 628.76 | $C_{33}H_{48}N_4O_8$ | 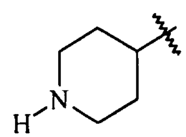 | H | 1.43 | 1.32 |

(continued)

| No. of compound | characteristics | MW | Molecular formula | $R_1$ | $R_2$ | Activity of HIV inhibition $IC_{50}$,μg/ml | Activity of HBV inhibition $IC_{50}$,μg/ml |
|---|---|---|---|---|---|---|---|
| GM-W38 | purple solid | 705.84 | $C_{38}H_{51}N_5O_8$ | 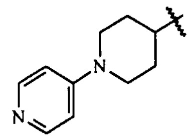 | H | 1.59 | 0.64 |
| GM-W39 | purple solid | 603.7 | $C_{31}H_{45}N_3O_9$ | 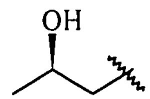 | H | 0.22 | 0.32 |
| GM-W40 | purple solid | 603.7 | $C_{31}H_{45}N_3O_9$ | 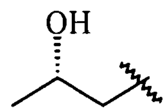 | H | 0.32 | 0.01 |
| GM-W41 | purple solid | 603.7 | $C_{31}H_{45}N_3O_9$ | 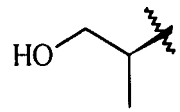 | H | 0.17 | 0.32 |
| GM-W42 | purple solid | 589.68 | $C_{30}H_{43}N_3O_9$ | 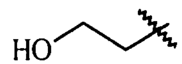 | H | 0.091 | 0.01 |
| GM-W43 | purple solid | 690.82 | $C_{35}H_{54}N_4O_{10}$ | 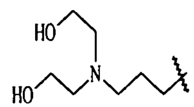 | H | 0.87 | 0.64 |
| GM-W44 | purple solid | 711.84 | $C_{41}H_{49}N_3O_8$ | 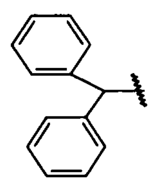 | H | >60 | >100 |

(continued)

| No. of compound | characteristics | MW | Molecular formula | $R_1$ | $R_2$ | Activity of HIV inhibition $IC_{50}$,μg/ml | Activity of HBV inhibition $IC_{50}$,μg/ml |
|---|---|---|---|---|---|---|---|
| GM-W45 | purple solid | 692.84 | $C_{38}H_{52}N_4O_8$ | 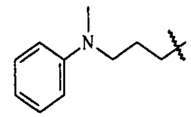 | H | <1.67 | 0.32 |
| GM-W46 | purple solid | 672.81 | $C_{35}H_{52}N_4O_9$ | 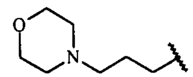 | H | 1.32 | 1.49 |
| GM-W47 | purple solid | 684.86 | $C_{37}H_{56}N_4O_8$ | 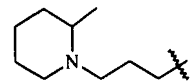 | H | 1.84 | 3.87 |
| GM-W48 | purple solid | 614.73 | $C_{32}H_{46}N_4O_8$ | 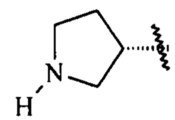 | H | 3.79 | 0.64 |
| GM-W49 | purple solid | 614.73 | $C_{32}H_{46}N_4O_8$ | 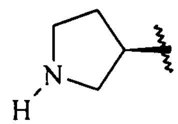 | H | 5.41 | 3.2 |
| GM-W50 | purple solid | 641.77 | $C_{33}H_{43}N_3O_8S$ | 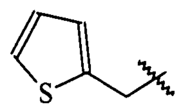 | H | 1.57 | 0.64 |
| GM-W51 | purple solid | 657.8 | $C_{34}H_{51}N_5O_8$ | 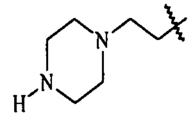 | H | 3.48 | 6.4 |
| GM-W52 | purple solid | 642.78 | $C_{34}H_{50}N_4O_8$ | 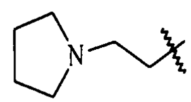 | H | 0.65 | 0.32 |

(continued)

| No. of compound | characteristics | MW | Molecular formula | $R_1$ | $R_2$ | Activity of HIV inhibition $IC_{50}$,μg/ml | Activity of HBV inhibition $IC_{50}$,μg/ml |
|---|---|---|---|---|---|---|---|
| GM-W53 | purple solid | 641.71 | $C_{33}H_{43}N_3O_{10}$ | 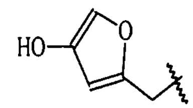 | H | 0.37 | 0.46 |
| GM-W54 | purple solid | 777.9 | $C_{45}H_{51}N_3O_9$ | 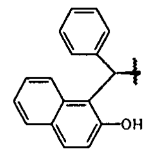 | H | >60 | >100 |
| GM-W55 | purple solid | 707.85 | $C_{39}H_{53}N_3O_9$ | 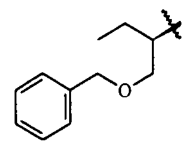 | H | 15.46 | 3.2 |
| GM-W56 | purple solid | 615.71 | $C_{32}H_{45}N_3O_9$ | 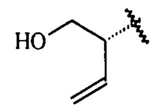 | H | 0.14 | 0.064 |
| GM-W57 | purple solid | 651.75 | $C_{34}H_{45}N_5O_8$ | 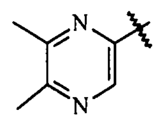 | H | 13.76 | 8.0 |
| GM-W58 | purple solid | 631.76 | $C_{33}H_{49}N_3O_9$ | 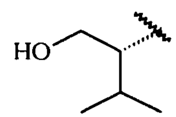 | H | 1.39 | 0.64 |
| GM-W59 | purple solid | 741.87 | $C_{42}H_{51}N_3O_9$ | 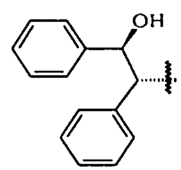 | H | >60 | 32 |

(continued)

| No. of compound | characteristics | MW | Molecular formula | $R_1$ | $R_2$ | Activity of HIV inhibition $IC_{50}$,μg/ml | Activity of HBV inhibition $IC_{50}$,μg/ml |
|---|---|---|---|---|---|---|---|
| GM-W60 | purple solid | 653.66 | $C_{30}H_{44}N_3O_{11}P$ | 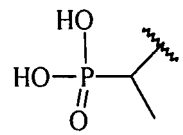 | H | 0.13 | 1.32 |
| GM-W61 | purple solid | 639.63 | $C_{29}H_{42}N_3O_{11}P$ | 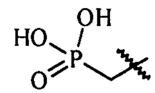 | H | 0.42 | 0.64 |
| GM-W62 | purple solid | 709.76 | $C_{34}H_{52}N_3O_{11}P$ | 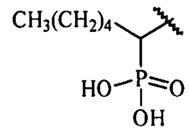 | H | 1.76 | 0.89 |
| GM-W63 | purple solid | 647.76 | $C_{33}H_{49}N_3O_{10}$ | 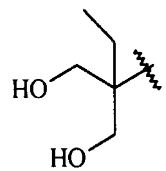 | H | 1.37 | 3.2 |
| GM-W64 | purple solid | 673.8 | $C_{34}H_{51}N_5O_9$ | 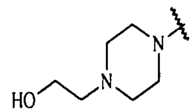 | H | 3.75 | 8.6 |
| GM-W65 | purple solid | 657.79 | $C_{35}H_{51}N_3O_9$ | 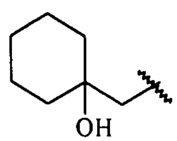 | H | 6.81 | 3.1 |

(continued)

| No. of compound | characteristics | MW | Molecular formula | $R_1$ | $R_2$ | Activity of HIV inhibition $IC_{50}$,μg/ml | Activity of HBV inhibition $IC_{50}$,μg/ml |
|---|---|---|---|---|---|---|---|
| GM-W66 | purple solid | 657.79 | $C_{35}H_{51}N_3O_9$ | 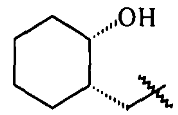 | H | 7.32 | 3.2 |
| GM-W67 | purple solid | 657.79 | $C_{35}H_{51}N_3O_9$ | 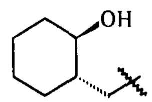 | H | 4.51 | 0.64 |
| GM-W68 | purple solid | 630.73 | $C_{32}H_{46}N_4O_9$ | 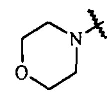 | H | 10.46 | 4.6 |
| GM-W69 | purple solid | 698.89 | $C_{38}H_{58}N_4O_8$ | 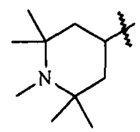 | H | 22.23 | 10.8 |
| GM-W70 | purple solid | 679.8 | $C_{37}H_{49}N_3O_9$ | 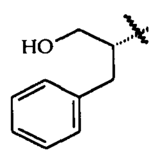 | H | 24.0 | 10.8 |
| GM-W71 | purple solid | 693.83 | $C_{38}H_{51}N_3O_9$ | 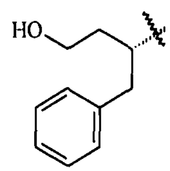 | H | 13.21 | 6.4 |
| GM-W72 | purple solid | 628.76 | $C_{33}H_{48}N_40_8$ | 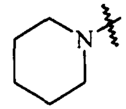 | H | 6.45 | 3.2 |

(continued)

| No. of compound | characteristics | MW | Molecular formula | $R_1$ | $R_2$ | Activity of HIV inhibition $IC_{50}$,μg/ml | Activity of HBV inhibition $IC_{50}$,μg/ml |
|---|---|---|---|---|---|---|---|
| GM-W73 | purple solid | 619.7 | $C_{31}H_{45}N_3O_{10}$ | 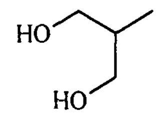 | H | 0.26 | 1.43 |
| GM-W74 | purple solid | 619.7 | $C_{31}H_{45}N_3O_{10}$ | 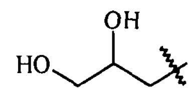 | H | 0.47 | 8.9 |
| GM-W75 | purple solid | 672.81 | $C_{35}H_{52}N_4O_9$ | 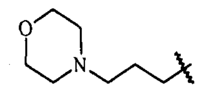 | H | 1.21 | 0.1 |
| GM-W76 | purple solid | 656.81 | $C_{35}H_{52}N_4O_8$ | 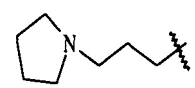 | H | 0.24 | 0.01 |
| GM-W77 | purple solid | 612.67 | $C_{30}H_{40}N_6O_8$ | 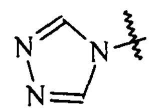 | H | 8.97 | 7.36 |
| GM-W78 | purple solid | 687.82 | $C_{36}H_{53}N_3O_{10}$ | 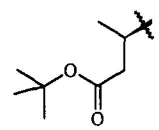 | H | 1.24 | 0.64 |
| GM-W79 | purple solid | 801.96 | $C_{42}H_{63}N_3O_{12}$ | 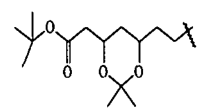 | H | 1.47 | 3.2 |
| GM-W80 | purple solid | 700.82 | $C_{36}H_{52}N_4O_{10}$ | 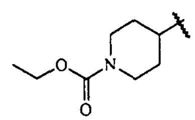 | H | 0.97 | 1.1 |

(continued)

| No. of compound | characteristics | MW | Molecular formula | $R_1$ | $R_2$ | Activity of HIV inhibition $IC_{50}$,μg/ml | Activity of HBV inhibition $IC_{50}$,μg/ml |
|---|---|---|---|---|---|---|---|
| GM-W81 | purple solid | 734.66 | $C_{36}H_{45}Cl_2N_3O_9$ | 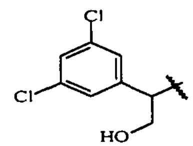 | H | 2.46 | 2.3 |
| GM-W82 | purple solid | 697.81 | $C_{37}H_{51}N_3O_{10}$ | 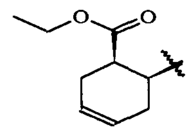 | H | 4.87 | 1.2 |
| GM-W83 | purple solid | 654.71 | $C_{33}H_{42}N_4O_{10}$ | 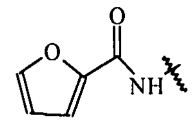 | H | 3.56 | 3.2 |
| GM-W84 | purple solid | 904.18 | $C_{52}H_{77}N_3O_{10}$ | 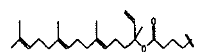 | H | 1.24 | 0.8 |
| GM-W85 | purple solid | 836.06 | $C_{47}H_{69}N_3O_{10}$ | 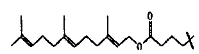 | H | 0.78 | 0.01 |
| GM-W86 | purple solid | 663.82 | $C_{33}H_{49}N_3O_9S$ | 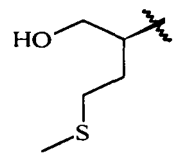 | H | 0.22 | 0.64 |
| GM-W87 | purple solid | 679.8 | $C_{37}H_{49}N_3O_9$ | 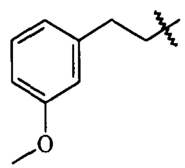 | H | 0.39 | 0.064 |

(continued)

| No. of compound | characteristics | MW | Molecular formula | $R_1$ | $R_2$ | Activity of HIV inhibition $IC_{50}$,μg/ml | Activity of HBV inhibition $IC_{50}$,μg/ml |
|---|---|---|---|---|---|---|---|
| GM-W88 | purple solid | 639.74 | $C_{34}H_{45}N_3O_9$ | 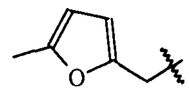 | H | 0.24 | 0.08 |
| GM-W89 | purple solid | 702.84 | $C_{39}H_{50}N_4O_8$ | 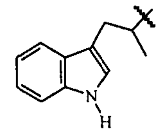 | H | 1.68 | 2.36 |
| GM-W90 | purple solid | | $C_{38}H_{48}N_4O_8$ | 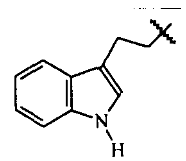 | H | 1.23 | 0.93 |

## Examples

The technicians in this art are expected to understand this invention more comprehensively by the following examples, however, none of which are intended to limit the scope of the invention.

Example 1 Preparation of 17-(2'-(1"-oxa-4"-azacyclohexyl-1"-)ethylamino)-17 -demethoxy geldanamycin (GM-APML)

50mg geldanamycin (89.29μmol) is added into 5mL $CHCl_3$ and 0.5ml methanol. The mixture is stirred until geldanamycin dissolved to result in an orange solution. 21mg (164μmol) 4-(2-aminoethyl)-1-oxa-4-azacyclohexane is subsequently added. After reacting at room temperature for 4 days, the solvent in resultant is evaporated to dryness to obtain dark purple solid. The solid residue is dissolved into 10mL ethyl acetate and the resulting solution is washed successively with deionized water, saturated $NaHCO_3$ solution, 1mol/L HCl solution and saturated NaCl solution. The organic phase is added with anhydrate $Na_2SO_4$ and is dried overnight. The $Na_2SO_4$ is then filtered out and the organic phase is concentrated under reduced pressure. The concentrated solution is then chromatographically separated using a silica gel column, 46.2mg GM-APML is then obtained (yield 61.2%).
$^1$H-NMR(400MHz,$CDCl_3$)δ(ppm):0.9-1.0(m,6H,C10-CH3,C14-CH3),1.28-1.38(m ,2H,C13-H2),1.5(m,1H,C14-H),1.64 (m,2H,C15-H2),1.78(s,3H,C8-CH3),2.03(s,3 H,C2-CH3),2.5(br,4H,C 17-NH-CH2-CH2-N-),2.6-2.7(br,4H,C17-N-CH2-CH2-O), 2.7-2.8(m,1H,C10-H),3.23(s,3H,C12-OCH3),3.36(s,3H,C6-OCH3),3.44(d,1H,J=9. 2Hz,C12-H),3.5(br, 1H,C17-NH),3.58(d,1H,J=9.2Hz,C11-H),3.64-3.8(m,4H,C170 -(CH2-CH2)-N-),4.31(d,1H,J=10.0Hz,C6-H),4.4(br,1H, C11-OH),4.84(br,2H,-NH2 ),5.19(s,1H,C7-H),5.83(t,1H,J=10.4,C5-H)5.86(d,1H,J=9.6,C9-H),6.58(t,1H,J=11. 2Hz,C4-H), 6.96(d,1H,J=11.6Hz,C3-H),7.15(br,1H,C20-NH-CO), 9.19(s,1H, C 19-H)

Example 2 Preparation of 17-(2'-(1"-azacyclohexyl-1"-)ethylamino)-17 -demethoxy geldanamycin(GM-AEPD)

GM-AEPD can be obtained according to the procedure similar to that used in Example 1 when the side chain reactant is 2-(1'-azacyclohexyl) ethylamine.
$^1$H-NMR(400MHz,$CDCl_3$)δ(ppm):0.82(1H,m,C14-H),0.94-1.0(m,6H,C10-CH3,C1 4-CH3),1.24-1.3(m,4H,C13-H2,C15-H2),1.4-1.5(m,2H,C17-N(CH2-CH2)$_2$CH2)), 1.6(br,4H,C17-N(CH2-CH2)$_2$CH2),1.76(br,1H,C10-H),1.78(s,3H,C8-CH3),2.03(s, 3H,C2-CH3),2.3-2.4(br,4H,C17-N-(CH2-CH2)$_2$CH2),2.6-2.8(m,4H,C17-NH-CH2 -CH2-N),3.24(s,3H, C12-OCH3),3.38(s,3H,C6-OCH3),3.44(d,1H,J=9.2Hz,C12-H), 3.58(d,1H,J=9.2Hz,C11-H),3.7(br,1H,C17-NH-),4.31(d, 1H,J=10.0Hz,C6-H),4.5(br ,1H,C11-OH),4.80(br,2H,-CO-NH2),5.20(s,1H,C7-H),5.83(t,1H,J=10.4,C5-H)5.94 (d,1H, J=9.6,C9-H),6.59(t,1H,J=11.6Hz,C4-H),6.96(d,1H,J=11.6Hz,C3-H),7.22(br, 1H,C20-NH-CO),9.19(s,1H, C 19-H)

Example 3 preparation of 17-(4'-benzyl-4'-azacyclohexyl amino)-17-demethoxy geldanamycin(GM-ABPD)

GM-ABPD can be obtained according to the procedure similar to that used in Example 1 when the side chain reactant is 4'-benzyl-4'-azacyclohexyl amine.
$^1$H-NMR(400MHz,$CDCl_3$)δ(ppm):0.94-1.0(dd,6H,C10-CH3,C14-CH3),1.5-1.6(m, 4H,C17-NH-CH(CH2-CH2)$_2$N-),1.64 (d,2H,C15-H2),1.7(m,2H,C13-H2),1.8(s,3H, C8-CH3),1.9(s,2H,C17-NH-CH-(CH$_2$-CH2)2-N-CH2-Ph),2.03(s,3H,C2-CH3),2.1-2.2(m,2H,C17-NH-CH-(CH2-CH2)$_2$-N-CH2-Ph),2.7-2.8(m,3H,C14-CH,C17-NH-CH-(CH2-CH2)$_2$-N-CH2-Ph),2.87(br,1H,C17-NH-CH(CH2-CH2)$_2$N-),3.26(s,3H, C 12-OCH3),3.38(s,3H,C6-OCH3),3.4(d,1H,J=9.2Hz,C12-H), 3.58(d,1H,J=9.2Hz,C 11-H),3.6(s,1H,C10-H),3.9(br,1H,C17-NH-),4.2(br,1H,C11-OH),4.3(d,1H,J=10.0H z,C6-H),4.78 (br,2H,-CO-NH2),5.17(s,1H,C7-H),5.8-5.9(m,2H,C5-H,C9-H),6.27(b r, 1H,C20-NH-CO),6.5(t,1H,J=11.2Hz,C4-H),6.9 (d,1H,J=11.6Hz,C3-H),7.34(br,5H ,C17-Ph),9.13(s,1H, C 19-H)

Example 4 preparation of 17-(tetrahydropiperazin-4'-yl-methylmino)-17-demethoxy geldanamycin(GM-AMPP)

GM-AMPP can be obtained according to the procedure similar to that used in Example 1 when the side chain reactant is tetrahydropiperazin-4'-yl-methylmine.
$^1$H- NMR (400MHz, DMSO) δ (ppm): 0.6 (d, 3H, C10- CH3), 0.8 (m, 3H, C14- CH3), 0.82-1.08 (m, 5H, C17-N-(CH2-CH2)$_2$-CH-CH2-NH2),1.24(m,2H,C13-H2),1.5(m,1H,C1 4-H),1.58(s,3H,C8-CH3),1.60(d,2H,C15-H2),1.8(s, 3H,C2-CH3),2.3(br,2H,C17-N-(CH2-CH2)$_2$-CH-CH2-NH2),2.4-2.5(br,4H,C17-N-(CH2-CH2)$_2$-),2.8(m,1H,C10-H),3.18 (br,6H,C6-OCH3,C12-OCH),3.28(d,1H,J=8.8Hz,C12-H),3.38(d,1H,J=8.8 Hz,C11-H),4.36(t,1H,C11-OH )4.82(d,1H, J=6.8Hz,C6-H), ,4.82(br,2H,-NH2),5.2( s,1H,C7-H),5.22(d,1H,J=10.0,C9-H),5.4(t,1H,J=10.4,C5-H),6.4(br,2H,C17-CH2-N H2),6.55(t,1H,J=11.2Hz,C4-H),6.99(br,1H,C20-NH-CO),7.1(s,1H,C19-H),7.12(d, 1H,J=11.0Hz,C3-H),7.4,7.7 (s,s,2H, -CO-NH2)

Example 5 preparation of 17-(myrtanylamino)-17-demethoxy geldanamycin (GM-MTA)

GM-MTA can be obtained according to the procedure similar to that used in Example 1 when the side chain reactant is myrtanylamine.
$^1$H-NMR(400MHz,$CDCl_3$)δ(ppm):0.87(d,3H,C14-CH3),0.95(d,3H,C10-CH3),1.02 (s,3H,MTA-9'CH3),1.24(s,3H,MTA-10'CH3),1.5-1.6(m,3H,MTA-5'CH2,2'-CH), 1.72(m,1H,C14-CH),1.80(s,3H,C8-CH3),1.8-2.0(m,6H,MTA-3'CH2,7'CH2,C15-CH2),2.02(s,3H,C2-CH3),2.32(m,1H,C17-6'CH),2.42(m,2H,C13-CH2),2.65(d,1H,

C10-CH),2.74(m,1H,C17-4'CH),3.374(s,3H,C12-OCH3),3.370(s,3H,C6-OCH3),3. 6-3.4(m,4H,C11-H,C12-H, C17-NH-CH2-),4.3(d,2H,J=10Hz,C6-H),4.37(br,1H,C1 1-OH),4.76(br,2H,C1-CO-NH2),5.20(s,1H,C7-H),5.857(t,1H, J=11.2Hz,C5-H),5.9 04(d,1H,J=10Hz,C9-H),6.38(br,1H,C20-NH-CO),6.58(t,1H,J=11.4Hz,C4-H),6.97( d,1H,J=11.6Hz, C3-H), 9.19(s,1H, C19-H)

Example 6 preparation of 17-diethyloxy phosphoryl methylene amino -17-demethoxy geldanamycin (GM-AP)

1.1g(3.4mmol) p-benzylsulfonyl methylene phosphonate diethyl ester is dissolved into 15ml DMF. 0.9g (4.8mmol) Phthalimide potassium salt is added into the resulting solution and mixed under stirring. The temperature of the mixture is raised gradually to 90˚C. The material disappears after reacting for 2h, then the mixture is cooled to room temperature. The solvent in the resultant is evaporated to dryness under reduced pressure. The residue is separated chromatographically using a silica gel column to obtain 500mg yellow solid product aminomethylene phosphonate diethyl ester- phthalimide.

200mg(0.7mmol) of the product obtained from the previous procedure is dissolved in 15ml ethanol, is added with 0.1ml (2mmol) hydrazine hydrate and is allowed to react for 4h at room temperature. The resultant is evaporated to dryness under reduced pressure, then ethyl acetate is added into the residue, mixed and the solid is filtered out. The filtrate is then separated using a silica gel column to obtain 80mg of colorless oily product aminomethylene phosphonate diethyl ester.

The product aminomethylene phosphonate diethyl ester is subsequently reacted with geldanamycin to obtain the product GM-AP according to the procedure similar to that used in Example 1.
$^1$H-NMR(300MHz,$CDCl_3$)δ(ppm):0.94-1.04(dd,6H,C10-CH3,C14-CH3),1.36(dt,6 H,-PO(OCH2CH3)$_2$),1.6-1.8(br,1H, C17-NH-CH2-),1.8(d,6H,C8-CH3,C2-CH3),2. 03(br,3H,C13-H2,C14-H),2.18(s,C10-H-),2.35(m,1H,C9-H),2.7(m,2H, C5-CH2), 3.28(s,3H,C12-OCH3),3.36(s,3H,C6-OCH3),3.43(d,1H,J=9.2Hz,C12-H),3.58(d,1 H,J=9.2Hz,C11-H),3.9-4.0 (dd,2H,C17-NH-CH2-P),4.15-4.2(five,4H,-PO(OCH2C H3)$_2$),4.3(d,1H,J=10.0Hz,C6-H),4.78(br,2H,-CO-NH2),5.27(s, 1H,C7-H),5.8(d,1H, C5-H),5.9(d,1H,C9-H),6.3(br,1H,C20-NH-CO),6.6(t,1H,J=11.2Hz,C4-H),6.9(d,1H ,J=11.6Hz,C3-H), 9.07(s,1H, C 19-H)

Example 7 preparation of 17-(3'-(1"-imidazolyl)propylamino)-17-demethoxy geldanamycin(ZJH061129)

ZJH061129 can be obtained according to the procedure similar to that used in Example 1 when the side chain reactant is *N*-(3-aminopropyl) imidazole.

ZJH061129 can be obtained according to the procedure similar to that used in Example 1 when the side chain reactant is N-(3-aminopropyl) imidazole.
$^1$H-NMR(400M, $CDCl_3$) δ(ppm): 0.87(d, 3H, J=6.5Hz, $CH_3$); 0.99(d, 3H, J=7.0Hz, $CH_3$); 1.64-1.72(m, 3H, CH, $CH_2$); 1.79(s, 3H, $CH_3$); 2.02(s, 3H, $CH_3$); 2.11-2.22(m, 3H, imidazole NCHa, $CH_2$); 2.62-2.65(m, 1H, OCH); 2.72-2.75(m, 1H, CH); 3.27(s, 3H, $OCH_3$); 3.35(s, 3H, $OCH_3$); 3.42-3.43(m, IH, OCH); 3.48-3.54(m, 3H, imidazole NCHb, $NCH_2$); 4.04-4.14 (m, 2H, $CH_2$); 4.28-4.31(m, 1H, OCH); 5.18(s, 1H, OCH); 5.84-5.88(m, 2H, 2×=CH); 6.19-6.21(m, 1H, Ar-H); 6.55-6.60 (m, 1H, =CH); 6.92-6.95(m, 1H, =CH); 7.11(s, 1H, Ar-H); 7.51(s, 1H, Ar-H); 9.12(s, 1H, Ar-H).
MS(ESI): m/z=654(M+1).

Example 8 preparation of 17-(4"-aminosulfonyl) phenylethylamino-17-demethoxy geldanamycin(ZJH061208)

ZJH061208 can be obtained according to the procedure similar to that used in Example 1 when the side chain reactant is 4-aminoethyl benzenesulfonamide.
$^1$H-NMR(400M, $CDCl_3$) δ(ppm): 0.94(d, 3H, $C_{14}$-$CH_3$); 1.00(d, 3H, $C_{10}$-$CH_3$); 1.24-1.31(m, 2H, $C_{13}$-$H_2$); 1.79(s, 3H, $C_8$-$CH_3$); 2.02(s, 3H, $C_2$-$CH_3$); 2.31-2.37(m, 1H, $C_{10}$-H); 2.66-2.69(m, 1H, $C_{11}$-H); 2.72-2.75(m, 1H, $C_{14}$-CH); 3.03-3.06 (m, 2H, $C_{24}$-$CH_2$); 3.27(s, 3H, $C_{12}$-$OCH_3$); 3.36(s, 3H, $C_6$-$OCH_3$); 3.43-3.58(m, 2H, $C_{15}$-$CH_2$); 3.76-3.86(m, 2H, $C_{25}$-$CH_2$); 4.11-4.13(m, 1H, $C_{12}$-CH); 4.31(d, 1H, $C_6$-CH); 5.19(s, 1H, $C_7$-CH); 5.84-5.89(m, 2H, $C_5$-CH, $C_9$-CH); 6.55-6.61(m, 1H, $C_4$-CH); 6.95(d, 1H, $C_3$-CH); 7.38(d, 2H, $C_{27}$-CH, $C_{31}$-CH); 7.91(d, 2H, $C_{28}$-CH, $C_{30}$-CH); 9.14(s, 1H, $C_{19}$-CH).
MS(ESI):m/z=767.0(M+K),751.0(M+Na).

Example 9 preparation of 17-(3',4'-(Methylenedioxy)benzylamino)-17- demethoxygeldanamycin(ZJH061217)

ZJH061217 can be obtained according to the procedure similar to that used in Example 1 when the side chain reactant is 3,4- (Methylenedioxy)benzylamine (piperonylamine).
$^1$H-NMR(400M, $CDCl_3$) δ(ppm):0.99-1.03(m, 6H, $2CH_3$); 1.80(s, 3H, $CH_3$); 2.03(s, 3H, $CH_3$); 2.41-2.47(m, 1H); 2.68(d, 1H); 2.73-2.77(m, 1H); 2.88(br, 1H); 2.95(br, 1H); 3.27(s, 3H, $OCH_3$); 3.37(s, 3H, $OCH_3$); 3.44-3.60(m, 2H, $CH_2$); 4.18 (br, 1H, OH); 4.31(d, 1H, J=10Hz); 4.48-4.68(m, 2H, $CH_2$); 4.79(br, 2H, $NH_2$); 5.19(s, 1H); 5.84-5.93(m, 2H, 2CH); 5.99 (d, 2H); 6.36(br, 1H); 6.58(t, 1H, J=11.5Hz); 6.73-6.82(m, 3H, 3CH); 6.96(d, 1H, J=12Hz); 7.30(s, 1H); 8.02(br, 1H); 9.16(s, 1H).
MS(ESI):m/z=718.2(M+K), 702.2(M+Na), 679.2($M^+$).

Example 10 preparation of 17-(2'-thienylethylamino)-17-demethoxy geldanamycin (ZJH061221)

ZJH061221 can be obtained according to the procedure similar to that used in Example 1 when the side chain reactant is 2-aminoethyl thiophene.
$^1$H-NMR(400M, $CDCl_3$) δ(ppm): 0.95-1.00(m, 6H, $2CH_3$); 1.79(s, 3H, $CH_3$); 2.02(s, 3H, $CH_3$); 2.35-2.41(m, 1H); 2.68-2.76 (m, 2H, 2CH); 2.95(s, 2H); 3.16-3.20(t, 2H, $CH_2$); 3.26(s, 3H, $OCH_3$); 3.36 (s, 3H, $OCH_3$); 3.43-3.58(m, 2H, $CH_2$); 3.76-3.84(m, 2H, $CH_2$); 4.30(d, 1H, J=10Hz); 4.84(br, 2H, $NH_2$); 5.18(s, 1H); 5.83-5.90(m, 2H, 2CH); 6.36(br, 1H, NH); 6.57(t, 1H, J=11.5Hz); 6.90-6.98(m, 3H, 3CH); 7.21(d, 1H, J=Hz); 7.61(d, 1H, J=15.5Hz); 8.01(br, 1H, OH); 9.14(s, 1H).
MS(ESI):m/z=678.2(M+Na), 656.2(M+H), 624.2(M-33, $-OCH_3$).

Example 11 preparation of 17-(trans-4'-hydroxyl cyclohexylamino)-17 -demethoxy geldanamycin(ZJH061223)

ZJH061223 can be obtained according to the procedure similar to that used in Example 1 when the side chain reactant is trans-4-amino cyclohexanol.
$^1$H-NMR(400M, $CDCl_3$) δ(ppm):0.97-1.01(m, 6H, $2CH_3$); 1.38-1.57(m, 2H, 2CH); 1.77(m, 1H, CH); 1.80(s, 5H, $CH_3$+ 2CH); 2.00(m, 2H, 2CH); 2.03(s, 3H, $CH_3$); 2.09-2.11(d, 2H, 2CH); 2.16-2.22(m, 1H); 2.72-2.77(m, 2H, 2CH); 3.27(s, 3H, $OCH_3$); 3.38(s, 3H, $OCH_3$); 3.45-3.60(m, 2H, $CH_2$); 3.72(m, 2H, $CH_2$); 3.88(m, 1H); 4.31(d, 1H, J=10Hz); 4.74(br, 2H, $NH_2$); 5.19(s, 1H); 5.84-5.92(m, 2H, 2CH); 6.25(br, 1H, NH); 6.58(t, 1H, J=11.5Hz); 6.94-7.00(m, 1H); 7.28(s, 1H); 9.17(s, 1H).
MS(ESI):m/z=667.3(M+Na), 644.3($M^+$).

Example 12 preparation of 17-(3'-(2"-pyrrolidonyl) propylamino)-17-demethoxy geldanamycin(ZJH061226)

ZJH061226 can be obtained according to the procedure similar to that used in Example 1 when the side chain reactant is N-(3'-aminopropyl)-2- pyrrolidone.
$^1$H-NMR(400M, $CDCl_3$) δ(ppm): 0.98-1.00(m, 6H, $2CH_3$); 1.25(s, 1H); 1.80(s, 3H, $CH_3$); 1.83-1.88(m, 2H, $CH_2$); 2.02(s, 3H, $CH_3$); 2.07(t, 2H, $CH_2$, J= Hz); 2.31-2.38(m, 1H); 2.42(t, 2H, $CH_2$, J= Hz); 2.66(d, 1H); 2.72-2.76(m, 1H); 3.26(s, 3H, $OCH_3$); 3.36 (s, 3H, $OCH_3$); 3.37-3.46(m, 5H, $2CH_2$+CH); 3.54-3.58(m, 3H, $CH_2$+ CH); 4.30(d, 1H, J=Hz); 4.80(br, 2H, $NH_2$); 5.18(s, 1H); 5.83-5.92(m, 2H, 2CH); 6.55-6.61(t, 1H, J= Hz); 6.72(br, 1H, NH); 6.95(d, 1H, J=Hz); 7.24(s, 1H); 7.26(s, 1H); 9.15(s, 1H);

Example 13 preparation of 17-(2"-(*N*-ethyl pyrrolidinyl)-methylamino)-17 -demethoxy geldanamycin(ZJH061228)

ZJH061228 can be obtained according to the procedure similar to that used in Example 1 when the side chain reactant is 2-aminomethyl -1-ethylpyrrole.
$^1$H-NMR(400M, $CDCl_3$) δ(ppm):0.96-1.01(m, 3H, $CH_3$); 1.09-1.14(s, 3H, $CH_3$); 1.54(s, 3H, $CH_3$); 1.50-1.52(m, 2H, $CH_2$); 1.70-1.82(m, 2H, $CH_2$); 1.81(s, 3H, CH3); 1.90-2.00(m, 1H, CH); 2.03(s, 3H, $CH_3$); 2.21-2.29(m, 2H, $CH_2$); 2.35-2.46 (m, 1H, CH); 2.65-2.80(m, 3H, $NCH_2$, NCH); 3.27(s, 3H, $OCH_3$); 3.37(s, 3H, $OCH_3$); 3.41-3.76(m, 4H, $2\times NCH_2$); 4.32 (d, 1H, J=10Hz, OCH); 4.51-4.20(m, 1H, OCH); 4.20-4.35(br); 5.19(s, 1H, OCH); 5.83-5.94(m, 2H, Ar-$CH_2$); 6.59(t, 1H, J=11.2Hz, =CH); 6.96(d, 1H, J=11.2Hz, =CH); 7.16-7.21(m, 1H, =CH); 7.26-7.32(m, 1H, =CH); 9.21-9.22(s,s,1H, Ar-H).
MS(ESI):m/z=657(M+1).

Example 14 preparation of 17-(2"*S*-2"-(*N*-ethylpyrrolidinyl)-methylamino)-17 -demethoxy geldanamycin(ZJH071206S)

ZJH071206S can be obtained according to the procedure similar to that used in Example 1 when the side chain reactant is (S)-2-aminomethyl-1-ethylpyrrole.
$^1$H-NMR(400M, $CDCl_3$) δ(ppm):0.82-0.89(m, 3H, $CH_3$); 0.95-1.00(m, 3H, $CH_3$); 1.08-1.14(m, 3H, $CH_3$); 1.25-1.30(m,

H,); 1.80(s, 3H, $CH_3$); 2.02(s, 3H, $CH_3$); 2.35-2.41(m, 1H); 2.68-2.76(m, 2H, 2CH); 2.95(s, 2H); 3.16-3.20(t, 2H, $CH_2$); 3.26(s, 3H, $OCH_3$); 3.36 (s, 3H, $OCH_3$); 3.43-3.58(m, 2H, $CH_2$); 3.76-3.84(m, 2H, $CH_2$); 4.30(d, 1H, J=10Hz); 4.84(br, 2H, $NH_2$); 5.18(s, 1H); 5.83-5.90(m, 2H, 2CH); 6.36(br, 1H, NH); 6.57(t, 1H, J=11.5Hz); 6.90-6.98(m, 3H, 3CH); 7.21 (d, 1H, J=Hz); 7.6 1 (d, 1H, J=15.5Hz); 8.01(br, 1H, OH); 9.14(s, 1H).
MS(ESI):m/z=678.2(M+Na), 656.2(M+H), 624.2(M-33, $-OCH_3$).

Example 15 preparation of 17-(2"R-2"-(N-ethylpyrrolidinyl)-methylamino)-17 -demethoxy geldanamycin(ZJH071210R)

ZJH071210R can be obtained according to the procedure similar to that used in Example 1 when the side chain reactant is (R)-2-aminomethyl-1-ethylpyrrole.
$^1$H-NMR(600M, $CDCl_3$) 8(ppm):0.83-0.89(m, 6H, $2CH_3$); 1.00()1.80(s, 3H, $CH_3$); 2.02(s, 3H, $CH_3$); 2.35-2.41(m, 1H); 2.68-2.76(m, 2H, 2CH); 2.95(s, 2H); 3.16-3.20(t, 2H, $CH_2$); 3.26(s, 3H, $OCH_3$); 3.36 (s, 3H, $OCH_3$); 3.43-3.58(m, 2H, $CH_2$); 3.76-3.84(m, 2H, $CH_2$); 4.30(d, 1H, J=10Hz); 4.84(br, 2H, $NH_2$); 5.18(s, 1H); 5.83-5.90(m, 2H, 2CH); 6.36(br, 1H, NH); 6.57(t, 1H, J=11.5Hz); 6.90-6.98(m, 3H, 3CH); 7.21(d, 1H, J=Hz); 7.61(d, 1H, J=15.5Hz); 8.01(br, 1H, OH); 9.14 (s, 1H).
MS(ESI):m/z=678.2(M+Na), 656.2(M+H), 624.2(M-33, $-OCH_3$).

Example 16 preparation of 17-(2'-(3", 4"-dimethylcaffeoyl amido)ethylamino)-17 -demethoxy geldanamycin(ZJH070413)

1.8g(0.01mol) caffeic acid is added into 15mL purified water and the resulted solution is adjusted to pH 13 using 30%NaOH to dissolve completely caffeic acid. 6g dimethyl sulfate (0.05mol) is added into the solution which is reacted at room temperature for 10h with stirring and adjusting pH to higher than 10 at intervals, then adjusting pH to 3 using 2N HCl. After filtering the resultant, the solid is washed with water until the water filtered out reaches a pH of 6-7. The solid is dried to obtain 3, 4-dimethyl caffeic acid.

5.25g ethylenediamine is added into a 250mL three necked flask, then 30mL 1,4-dioxane is added and stirred. To the flask the solution of 2.45g di-*tert*-butyl carbonate in 30mL 1,4-dioxane is added dropwise at room temperature and under nitrogen protection. After reacting for 2h, the resultant is evaporated to dryness under reduced pressure. 50mL purified water is added into the residues under stirring and white solid precipitate can be seen. The precipitate is filtered and washed with water. The filtrate is extracted 3 times with 50mL methylene chloride. The extractants are pooled and dried on anhydrous sodium sulfate, then filtered. The filtrate is evaporated to dryness to obtain colorless oily liquid. The product is separated chromatographically with a silica gel column to obtain mono-*N*-*tert*-butyloxycarbonylethylenediamine.

0.208g(0.001mol) 3,4-dimethyl caffeic acid is added into 3mL dichlorosulfoxide and the mixture is reacted at 50°C for 4h. The resultant is evaporated into dryness under reduced pressure using an aspirator pump. 5mL methylene dichloride is subsequently added to the residues and, the mixture is stirred. The solution of 0.160g mono- *N*-*tert*-butyloxycarbonyldiamino ethane in 4mL pyridine is added to the mixture and the resulted mixture is allowed to react for 3h at room temperature. The resultant is filtered and the filtrate is washed successively with saturated $NaHCO_3$ solution and water. Then it is dried on anhydrous sodium sulfate and subsequently filtered. The filtrate is evaporated to dryness and separated chromatographically using a silica gel column to obtain (2-*tert*-butoxycarbonylamino)ethyl-3, 4-dimethyl caffeoylamide.

2mL methanol is added into 3-necked flask placed in an ice bath and 1mL acetyl chloride is added dropwise into it. The mixture is subsequently stirred to react at room temperature for 30 min. The methanol solution of 0.263g (0.75mmol) (2-*tert*-butoxycarbonyl amino)ethyl-3, 4-dimethyl caffeoylamide is added dropwise into the resultant and the resulted mixture is allowed to react completely at room temperature for 3h, The resultant is filtered and washed with methanol. The filtrate is evaporated to dryness under reduced pressure and is added with petroleum ether to precipitate yellow solid. The latter is filtered out and washed successively with ethyl acetate and chloroform. The resulted solid is dried over heat to obtain (2-amino)ethyl-3,4-di-hydroxyl-methylated caffeoyl amide hydrochloride.

50mg(89.29$\mu$mol) geldanamycin is added into 5mL $CHCl_3$ and 0.5mL methanol and the mixture is stirred until the geldanamycin dissolved to form an orange solution. 75mg(260$\mu$mol) of the N-aminoethyl- 3,4-dimethylated caffeoylamide hydrochloride produced with the previous procedure and 0.5mL triethylamine are added into the solution. The mixture is allowed to react for 3 days at room temperature and the solvent in it is evaporated to dryness to obtain purple solid. The product is separated chromatographically using a silica gel column to obtain 55.2mg 17-(2'-(3",4"-dimethylated caffeoylamido) ethylamino)-17-de-methoxy geldanamycin (ZJH070413) (79.4%).
$^1$H-NMR(500M, $CDCl_3$) $\delta$(ppm):0.98(m, 6H, $2CH_3$); 1.80(s, 3H, $CH_3$); 2.02(s, 3H, $CH_3$); 2.37-2.42(m, 1H); 2.65(d, 1H); 2.72-2.76(m, 1H); 3.07-3.12(m, 2H, $CH_2$); 3.26(s, 3H, $OCH_3$); 3.35(s, 3H, $OCH_3$); 3.57-3.58(m, 2H, $CH_2$); 3.71-3.85(m, 2H, $CH_2$); 3.90(s, 6H, $2CH_3$); 4.25(br, 1H, OH); 4.30(d, 1H, J=10Hz); 4.80(br, 1H, NH); 5.18(s, 1H); 5.84-5.90(m, 2H, 2CH); 6.15-6.17(m, 1H); 6.30(d, 1H, J=15.5Hz); 6.57(t, 1H, J=11.5Hz); 6.83-6.84(m, 1H); 6.86(d, 1H, J=8Hz); 6.94(d, 1H, J=12Hz); 7.02(s, 1H); 7.08(d, 1H, J=8Hz); 7.24(s, 1H); 7.61(d, 1H, J=15.5Hz); 9.13(s, 1H); 12.00(br, 4H,CONH).

Example 17 preparation of 17-(2'-nicotinamidoethylamino)-17-demethoxy geldanamycin(ZJH070418)

Mono-*N*-*tert*-butoxycarbonyldiamino ethane can be prepared according to the procedure provided in Example 16.

1.85g(0.015mol) nicotinic acid is added into 5mL methylene chloride under stirring and it is not dissolved. 4.4mL (0.06mol) dichlorosulfoxide is added into the mixture and the resulted mixture is allowed to react for 4h at 50˚C under nitrogen protection and with refluxing in a oil bath. Then the oil bath is removed and the resultant is filtered. The solid residue is washed with methylene chloride to obtain nicotinoyl chloride as white acicular crystals.

2.4g mono-*N*-*tert*-butoxycarbonyldiamino ethane (0.015mol) is added into 2ml methylene chloride and 2mL tetrahydrofuran. With stirring, 5mL triethylamine and the solid nicotinoyl chloride obtained in the previous procedure are successively added. The mixture is allowed to react completely at room temperature for 3h. Then the resultant is filtered and subsequently washed with methylene chloride to obtain a viscous liquid. The product is separate chromatographically using a silica gel column to obtain (2-*tert*-butoxycarbonylamino)ethyl nicotinylamide.

4ml anhydrous methanol is added into 3-necked flask placed in an ice bath, then 2mL acetyl chloride is added dropwise. The mixture is allowed to react subsequently at room temperature for 30 min. 0.53g(2mmol) (2-*tert*-butoxycarbonylamino)ethyl nicotinylamide solution in methanol is added into the resultant and the resulted mixture is allowed to react completely at room temperature for 30 min. After filtering and washing the resultant with ethyl acetate, the white solid obtained is (2-amino) ethyl nicotinylamide.

50mg geldanamycin (89.29μmol) is added into 5mL $CHCl_3$ and 0.5mL methanol, then geldanamycin is dissolved with stirring to make the orange reactive solution. 44mg (2-amino)ethyl nicotinylamide (153μmol) obtained from the previous procedure and 0.5ml triethylaime is added into said reactive solution. The resulted mixture is allowed to react at room temperature for 2 days, then the resultant solution is evaporated to dryness to obtain purple solid. The product is separated chromatographically using a silica gel column to obtain 58.3mg (94.2%) of 17-(2'-nicotinylamioethylamino)-17-demethoxy geldanamycin (ZJH070418).

$^1$H-NMR (500M, $CDCl_3$) δ(ppm):0.98-0.99(m, 6H, $2CH_3$); 1.80(s, 3H, $CH_3$); 2.02(s, 3H, $CH_3$); 2.42-2.46(m, 1H); 2.65(d, 1H); 2.72-2.76(m, 1H); 3.09-3.12(m, 2H, $CH_2$); 3.27(s, 3H, $OCH_3$); 3.35(s, 3H, $OCH_3$); 3.42-3.57(m, 2H, $CH_2$); 3.79-3.93 (m, 4H, $2CH_2$); 4.30(d, 1H, J=10Hz); 4.80(br, 2H, $NH_2$); 5.17(s, 1H); 5.30(br, 1H); 5.84-5.90(m, 2H, 2CH); 6.57(t, 1H, J=11.5Hz); 6.93-6.95(d, H, CH); 7.21(s, 1H); 7.53 (s, 1H); 8.44(d, 1H, J=15.5Hz); 8.76 (s, 1H); 9.13(s, 1H); 9.34(s, 1H); 11.89(br, 3H, 3NH).

Example 18 preparation of 17-(4'-((5"-(4"'-amino-2"'-oxopyrimidine -1"'-(2*H*)-yl)-3",4"-dihydroxyl-tetrahydrofuran-2"yl) methoxyl)-4'-oxobutylamino) -17 -de methoxy geldanamycin(GM-CY)

The primary amino group of the γ-aminobutyric acid is protected with $Boc_2O$ to obtain γ-*tert*-butoxycarbonylamino butyric acid according to the literature (Zhao Zhizhong et al. Protecting Groups in Organic Chemistry, Science Press, 1984: 41-49).

0.476g *p*- toluenesulfonic acid (2.5mmol) is added into 10mL acetone. After dissolution of the solid, 1.5mL 2,2-dimethoxy propane (12mmol) and 0.486g cytidine (2mmol) are further added into the solution. The mixture is allowed to react with stirring at room temperature for 1.5h. The reaction produce is large amount of white solid, which is filtered out and dried over heat to obtain 2', 3'-isopropylidenecytidine *p*- toluenesulfonate, which is reserved for further synthesis.

0.457g γ-*tert*-butoxycarbonylaminobutyric acid (2.25mmol) is added into 5mL $CHCl_3$. After dissolving, 0.6g dicyclohexylcarbodiimide (DCC) (2.91mmol) is added into the solution with stirring at room temperature, white precipitate appears in the solution. After reacting for 4h, the white precipitate is filtered out and the collected filtrate containing γ-butoxycarbonylaminobutyric anhydride is reserved for further synthesis.

Isopropylidenecytidine *p*- toluenesulfonate is placed into a 100mL round-bottom flask. 15mL methylene chloride and 1mL triethylamine are added into the flask, then the mixture is stirred until the solid dissolved. The filtrate from the previous synthesis is transferred into the flask. The resulted mixture is reacted under nitrogen protection for 30h with stirring, then the insoluble materials are filtered out. The resultant filtrate is condensed under reduced pressure with a vacuum oil pump to obtain yellowish viscous liquid, which is separated chromatographically with a silica gel column to obtain esterification product of 2',3'-isopropylidenecytidine with γ-butoxycarbonylaminobutyric acid.

4ml anhydrous methanol is added into a three-necked flask, cooled in an ice bath and 2mL acetyl chloride is added dropwise into the flask. The mixture is allowed to react for 30 min after completion of the dropping. Methanol solution of 50mg (0.107mmol) the esterification product of 2',3'-isopropylidenecytidine with γ-butoxycarbonylaminobutyric acid is added into the flask and is allowed to react completely for 30 min at room temperature. The resultant is filtered and the solid is washed with ethyl acetate to obtain white solid cytidine γ-amino butyrate hydrochloride.

50mg geldanamycin (89.29μmol) is added into 5mL $CHCl_3$ and methanol 0.5ml, then the mixture is stirred until geldanamycin dissolved and the color of the liquid turns orange. 80.2mg cytidine γ-amino butyrate hydrochloride (200μmol) is added into the orange liquid and the resulted mixture is allowed to react for 3 days at room temperature.

The solvent in resultant is evaporated to dryness to obtain dark purple solid. The solid residue is dissolved into 10mL ethyl acetate and is washed successively with deionized water, saturated $NaHCO_3$ solution, 1mol/L HCl solution and saturated NaCl solution. The organic phase is dried overnight on anhydrous $Na_2SO_4$. Then the anhydrous $Na_2SO_4$ is filtered out and the organic phase is concentrated under reduced pressure. The product is separated chromatographically using a silica gel column to obtain 17-(4'-((5"-(4"'-amino-2"'-oxopyrimidine-1"'-(2*H*)-yl)-3",4"-dihydroxyl-tetrahydrofuran -2"-yl) methoxy)-4'-oxobutylamino)-17-demethoxy geldanamycin.
$^1$H-NMR(400M, $CDCl_3$) δ(ppm):0.94-1.00(m, 6H, $2CH_3$); 1.24-1.30(m, 2H,$CH_2$); 1.64-1.67(m, 2H, $CH_2$); 1.80(s, 3H, $CH_3$); 2.02(s, 3H, $CH_3$); 2.38(t, 2H, $CH_2$); 2.41-2.47(m, 1H); 2.66-2.75 (m, 1H); 2.72-2.76(m, 1H); 2.98(t, 2H, $CH_2$); 3.27 (s, 3H, $OCH_3$); 3.37(s, 3H, $OCH_3$); 3.42-3.59(m, 3H, CH+$CH_2$); 3.62-3.66(m, 1H); 3.78-3.81(m, 1H); 3.89-3.94(m, 2H, $CH_2$); 4.08-4.11 (m, 1H); 4.31 (d, 1H); 4.81 (br, 2H, $NH_2$); 4.95(br, 1H, OH); 5.19(s, 1H); 5.26(br, 1H, OH); 5.69(d, 1H); 5.75(d, 1H); 5.84-5.90(m, 2H, 2CH); 6.55-6.61 (m, 1H); 6.93-6.95(d, 1H); 7.11(br, 2H, $NH_2$); 7.28(s, 1H); 7.82(d, 1H); 9.14(br, 1H, NH).
MS(ESI):m/z=857.3($M^+$), 880.3(M+Na).

Example 19 preparation of 2'R-17-tetrahydrofurfurylamino- 17-demethoxy geldanamycin(THFM(R)-GM)

THFM(R)-GM can be obtained according to the procedure similar to that used in Example 1 when the side chain reactant is (*R*)-tetrahydrofurfurylamine.
$^1$H-NMR(400M, $CDCl_3$) δ(ppm):0.9-1.0(m, 6H, $2CH_3$); 1.25(s, 2H, $CH_2$); 1.4-1.5(m, 1H); 1.61-1.65(m, 2H, $CH_2$); 1.70-1.74 (m, 2H,$CH_2$); 1.799(s, 3H, $CH_3$); 1.93-1.98(m, 2H, $CH_2$); 2.025(s, 3H, $CH_3$); 2.36-2.39(m, 1H); 2.66-2.75 (m, 2H, $CH_2$); 3.268(s, 3H, $OCH_3$); 3.362(s, 3H, $OCH_3$); 3.42-3.49(m, 1H); 3.56-3.62(m, 1H); 3.79-3.95 (m, 2H, $CH_2$); 4.08-4.11 (m, 1H); 4.311 (d, 1H); 4.806(br, 2H, $NH_2$); 5.190(s, 1H); 5.857(t, 1H); 5.904(d, 1H); 6.583(t, 1H); 6.955(d, 1H); 7.276(s, 1H); 9.167(br, 1H, NH).
MS(FAB):m/z=631 (M+1).

Example 20 preparation of 2'S-17-tetrahydrofurfurylamino-17-demethoxy geldanamycin (THFM(*S*)-GM)

THFM(*S*)-GM can be obtained according to the procedure similar to that used in Example 1 when the side chain reactant is (*S*)-tetrahydrofurfurylamine.

The retention time of THFM(S)-GM differs minutely from that of THFM(R)-GM in the HPLC grams, the $^1$H-NMR spectra of both compounds are essentially same.
$^1$H-NMR(400M, $CDCl_3$) δ(ppm):0.94-1.00(m, 6H, $2CH_3$); 1.25(s, 2H, $CH_2$); 1.30-1.32(m, 1H); 1.61-1.64(m, 2H, $CH_2$); 1.73-1.75(m, 2H, $CH_2$); 1.80(s, 3H, $CH_3$); 1.93-2.00(m, 2H, $CH_2$); 2.03(s, 3H, $CH_3$); 2.31-2.37(m, 1H); 2.67-2.75 (m, 2H, $CH_2$); 3.27(s, 3H, $OCH_3$); 3.36(s, 3H, $OCH_3$); 3.43-3.49(m, 1H); 3.58-3.62(m, 1H); 3.79-3.96(m, 2H, $CH_2$); 4.08-4.11 (m, 1H); 4.31(d, 1H); 4.81(br, 2H, $NH_2$); 5.19(s, 1H); 5.86(t, 1H); 5.91(d, 1H); 6.55-6.60(m, 1H); 6.95(d, 1H); 7.28(s, 1H); 9.14(br, 1H, NH).
MS(FAB):m/z=631 (M+1).

Example 21 preparation of 17-tetrahydrofurfurylamino-17-demethoxy geldanamycin (THFM-GM)

THFM-GM can be obtained according to the procedure similar to that used in Example 1 when the side chain reactant is tetrahydrofurfurylamine.
$^1$H-NMR(400M,$CDCl_3$)δ(ppm):0.90-1.01(m,6H,$C_{10}$-$CH_3$,$C_{14}$-$CH_3$);1.25(s,2H,$C_{13}$-$H_2$);1.4-1.5(m,1H,$C_{14}$-H);1.61-1.65 (m,2H,THMF-$CH_2$-C$\underline{H_2}$-$CH_2$-);1.70-1.74(m,2H ,$C_{15}$-$H_2$);1.79(s,3H,$C_8$-$CH_3$);1.93-1.98(m,2H,THMF-$CH_2$-C$\underline{H_2}$-CH-); 2.02(s,3H,$C_2$-$CH_3$);2.36-2.39(m,1H,$C_{10}$-H);2.66-2.75(m,2H,$C_{17}$-NH-C$\underline{H_2}$-);3.26(s,3H,$C_{12}$-$OCH_3$); 3.36(s,3H, $C_6$-$OCH_3$);3.42-3.49(m,1H,$C_{12}$-H);3.56-3.62(m,1H,$C_{11}$-H);3.79-3.95(m, 2H,THMF-$CH_2$-C$\underline{H_2}$-O);4.08-4.11(m,1H, $C_{17}$-NH-$CH_2$-C$\underline{H}$-);4.31(d,J=10Hz,1H,$C_6$ -H); 4.80(br,OH,NH);5.19(s,1H,$C_7$-H);5.85(t,J=11.2Hz,1H,$C_5$-H);5.90(d, J=10Hz,1H ,$C_9$-H);6.58(t,J=11.4Hz,1H,$C_4$-H);6.95(d,J=11.6Hz,1H,$C_3$-H);7.27(s,1H,$C_{19}$-H);9.16( s,1H,CH).MS(FAB): m/z=654(M+Na).

Example 22 preparation of 17, 19-di-(*R*)-tetrahydrofurfuryl amino-17-demethoxy geldanamycin(THFM- II)

THFM- II can be obtained according to the procedure similar to that used in Example 1 when the side chain reactant is (R)- tetrahydrofurfurylamine. In this case the amount of the side chain compound fed is increased five fold and the reaction time elongated to 10h.
$^1$H-NMR(400M, $CD_3COD$) δ(ppm):0.73(d, 3H, J=6.4Hz, $CH_3$); 1.02(d, 3H, J=6.8Hz, $CH_3$); 1.46-1.65(m, 2H, $CH_2$); 1.60 (s, 3H, $CH_3$); 1.81-2.04(m, 5H, CH, $2CH_2$); 1.91(s, 3H, $CH_3$); 2.28-2.46(m, 2H, $CH_2$); 2.56-2.66(m, 2H, $CH_2$); 3.08-3.17 (m, 1H, CH); 3.23(s, 3H, $OCH_3$); 3.29(s, 3H, $OCH_3$); 3.44-3.88(m, 11H, 3OCH, $2OCH_2$, $2NCH_2$); 4.00-4.09(m, 2H, $CH_2$);

4.34-4.38(dd, 1H, $J_1$=10Hz, $J_2$=7.0Hz, OCH); 4.88(d, 1H, J=4.8Hz, OCH); 5.27(s, 1H, OCH); 5.29(d, 1H, J=10Hz, =CH); 5.49(t, 1H, J=10Hz, =CH); 6.57(t, 1H, J=12Hz, =CH); 7.27(d, 1H, J=12Hz, =CH).
MS(+Q1):m/z=753($M^+$+Na), 731($M^+$+1).

Example 23 preparation of 17, 19-di-(*S*)-tetrahydrofurfuryl amino-17-demethoxy geldanamycin (THFM+2)

The product THFM+2 can be synthesized according to the procedure similar to that used in Example 1 when the side chain reactant is (*S*)-tetrahydrofurfurylamine. In this case the amount of the side chain compound fed is increased five fold and the reaction time elongated to 10h.
$^1$H-NMR(400M, $CD_3COD$) δ(ppm):0.73(d, 3H, J=6.4Hz, $CH_3$); 0.92(d, 3H, $CH_3$); 1.45-1.59 (m, 2H, $CH_2$); 1.60(s, 3H, $CH_3$); 1.83-2.02(m, 5H, CH, $2CH_2$); 1.90(s, 3H, $CH_3$); 2.29-2.46(m, 2H, $CH_2$); 2.56-2.65(m, 2H, $CH_2$); 3.07-3.12(m, 1H, CH); 3.24(s, 3H, $OCH_3$); 3.29(s, 3H, $OCH_3$); 3.47-3.85(m, 11H, 3OCH, $2OCH_2$, $2NCH_2$); 4.00- 4.08(m, 2H, $CH_2$); 4.34-4.38(dd, 1H, $J_1$=10Hz, $J_2$=7.0Hz, OCH); 4.88(d, 1H, J=4.8Hz, OCH); 5.26(s, 1H, OCH); 5.29(d, 1H, J=10Hz, =CH); 5.47(t, 1H, J=10Hz, =CH); 6.58(t, 1H, J=12Hz, =CH); 7.24(d, 1H, J=12Hz, =CH).
MS(+Q1):m/z=753(M+Na), 731(M+1).

Example 24 Test procedure for herpes simplex virus activity (VR733 strain)

0.1 ml 0.25% trypsin solution and 5 ml 0.02% EDTA solution are added into a culture flask confluent with VERO cells. The culture is digested 20-25 min at 37˚C and the digestion liquid is discarded. Then the cells are dispersed with adding culture medium and passage at a ratio of 1:3. The cells reach confluence after 3 days of culture. The culture is prepared into a concentration of 200,000-300,000 cells/mL and is inoculated into 96 well culture plate in 0.1ml each well. The cells are cultured at 37˚C under 5%$CO_2$ condition for 24h. Tests are carried out when the cells grow into a mono layer.

Cell culture with a concentration of 200,000-300,000 VERO cells/mL is inoculated into 96 well culture plate in 0.1ml each well. The cells are cultured at 37˚C and under 5%$CO_2$ condition for 24h, then the culture medium is discarded. Appropriate amount of HSV-1 is added into the culture plate and the virus is allowed to absorb for 1h, then the virus liquid is discarded. The reagents to be tested (i.e target compounds of this invention) are added into the culture plate, wherein a series of concentrations of the reagent to be tested is added into culture plate in 2 wells per one concentration. The cells are cultured at 37˚C in 5%$CO_2$ and the pathological changes of the cells are observed after culturing for 48h. It is calculated that the effective concentration of the test reagent to inhibit 1/2 virus according to the following equation:

$$IC_{50} = Anti\log\left( A + \frac{50-A}{B-A} \times C \right)$$

*A* = log(pathological changes<50%concentration of the reagent)
*B* = log(pathological changes>50%concentration of the reagent)
*C* = log(dilution factor)

The calculated $IC_{50}$ values according to the test results are shown in Table 1.

Example 26 Test procedure for anti-HBV activity

Using cell culture method, the preparation of the cells to be tested (100,000 cells/mL) is inoculated into cell culture plate in 100μl each well and the cells are cultured 24h at 37˚C in 5%$CO_2$. Tests are carried out when the cells grow into a monolayer. The target compounds as well as the controls are prepared into a series of solutions of appropriate concentrations using the culture medium and are added into 96 well culture plate respectively in 4 wells per one concentration. Then the reagent solution in each well is changed into the fresh reagent solution with the same concentration every 4 days. A cell control without reagent treatment is simultaneously is set up. The observation index is based upon the pathological changes of the cells by observing the degrees of the pathological changes of the cells under microscope every 8 days. The test procedure for testing the inhibition of HBV activity of the drug is as follows: the tested cells at a concentration of 100,000/mL are inoculated into 96 well culture plate in 100μl each well and are cultured at 37˚C in 5%$CO_2$ for 24h, then the reagent solutions are added into the well. Cell control without drug treatment is simultaneously is set up. The reagent solution or control medium in each well is changed into the fresh reagent solution or fresh medium respectively every 4 days. After cytolysis of the cells, HBV DNA is extracted from the cell lysis solution according to the

molecular cloning technical procedure. The spots of different samples are hybridized and the A values of different hybridized spots are measured using autoradiograghic technique. The HBV DNA contents in the cell control as well as in the drug treated samples are calculated using the regression equations obtained from the standard curves to obtain half effective concentration values, the results are shown in Table 1.

Example 27 Test procedure of anti HIV-1 activity

8 reagent solutions of different diluted concentrations and positive control solutions are added into cell cultures in 96 well plate respectively. The sample of each diluted solutions is made duplicately and a control cell sample is also is set up. 100μl of cell sample at a concentration of $2\times10^5$cells/ml is inoculated into the wells containing reagent in the plate. The cells samples are cultured in a saturated humidity culture chamber (at a 5%$CO_2$ atmosphere) at 37°C. The pathological changes of cells are observed daily. The contents of HIV-1 P24 antigen in the cell cultures are measured at 4 days after addition of the reagents according to the procedure provided by the HIV-1 P24 antigen test kit, the half effective concentrations ($IC_{50}$) of the reagents are calculated, the results are shown in Table

## Claims

1. A series of geldanamycin derivatives whose structure is shown in Formula (I):

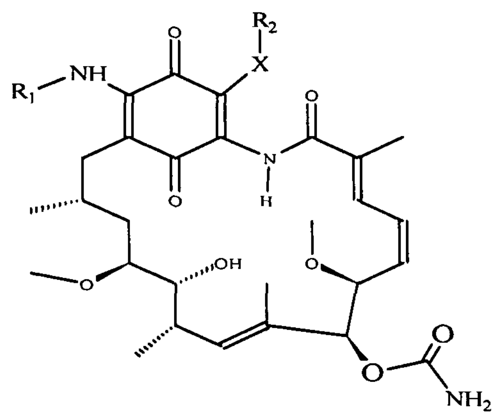

(I)

Wherein
$R_1$ is a substituent which has a linkage moiety on its one end consisting of linear or branched, saturated or unsaturated chain containing 3 to 20 carbon atoms and containing or not containing ether or ester or amide bonds in said chain, and the other end of the substituent is an alicyclic or an aromatic cyclic group which may optionally be substituted by hydrocarbyl, halogen, hydroxyl, carboxyl, nitrile group, amino, sulfonic or phosphoric acid group or esters or salts thereof;
$R_2$ is H or a same substituent as $R_1$ or a different substituent from $R_1$.
X is NH, O or S; or X-$R_2$ is H.

2. A method for preparing the geldanamycin derivatives defined in claim 1, wherein the amine containing $R_1$ substituent is allowed to react with geldanamycin in a haloalkane, alcoholic or polar aprotic solvent and under alkaline condition to obtain 17-mono-substituted compound (Formula I, wherein X-$R_2$ is H); then the resulting 17-mono-substituted compound is allowed to react with $R_2$XH under similar conditions to obtain 17,19-disubstituted compounds (Formula I, both $R_1$ and X-$R_2$ are not H).

3. The method of claim 2, wherein said solvent is selected from a group consisting of N, *N*-dimethylformamide, dimethylsulfoxide, ethyl acetate, acetonitrile and acetone.

4. The method of claim 2, wherein the alkaline condition is realized by using triethylamine, pyridine, *N*, *N*-dimethylpridine,

potassium carbonate, sodium carbonate or calcium hydroxide.

**5.** A method for preparing the geldanamycin derivatives defined in claim 1, wherein, when $R_1$ is a substituent containing a structure of 3, 4-di-hydroxyl-methylated caffeic acid, the method is as follows, caffeic acid is firstly reacted with methylating reagent under alkaline condition to obtain 3,4-di-hydroxyl-methylated caffeic acid, which reacts subsequently with acyl chlorinating reagent to obtain the corresponding acyl chloride, which further reacts with mono *N-tert*-butoxycarbonylethyldiamine to obtain (2-*tert*-butoxycarbonylamino)ethyl-3,4-di-hydroxyl-methylated caffeoylmide, after removal of *tert*-butyl protecting group to obtain (2-amino)ethyl-3,4-di-hydroxyl-methylated caffeoylamide, which further reacts with geldanamycin according to the method of claim 2 to obtain geldanamycin derivative containing di-hydroxyl-methylated caffeoylamido structure linked to the 17-site.

**6.** The method of claim 5, wherein the methylating reagent is selected from a group consisting of dimethyl sulfate, methyl methanesulfonate, methyl iodide and dimethyl carbonate.

**7.** A method for preparing the geldanamycin derivatives defined in claim 1, wherein, when $R_1$ is a substituent containing a structure of cytidine, the method is as follows, cytidine reacts with 2,2-dimethoxylpropane under acidic condition to obtain 2',3'-isopropylidene cytidine, which condensates with γ-*tert*-Butoxycarbonylamino butyric acid under the effect of dehydrating reagent DCC or TBU to obtain esterification product of said acid with 2', 3'-isopropylidene cytidine, from which removes the BOC protective group by alcoholysis under acidic catalysis to obtain cytidine γ-aminobutyrate hydrochloride, which reacts with geldanamycin according to the method of claim 2 to obtain geldanamycin derivative containing cytidine structure linked to the 17-site.

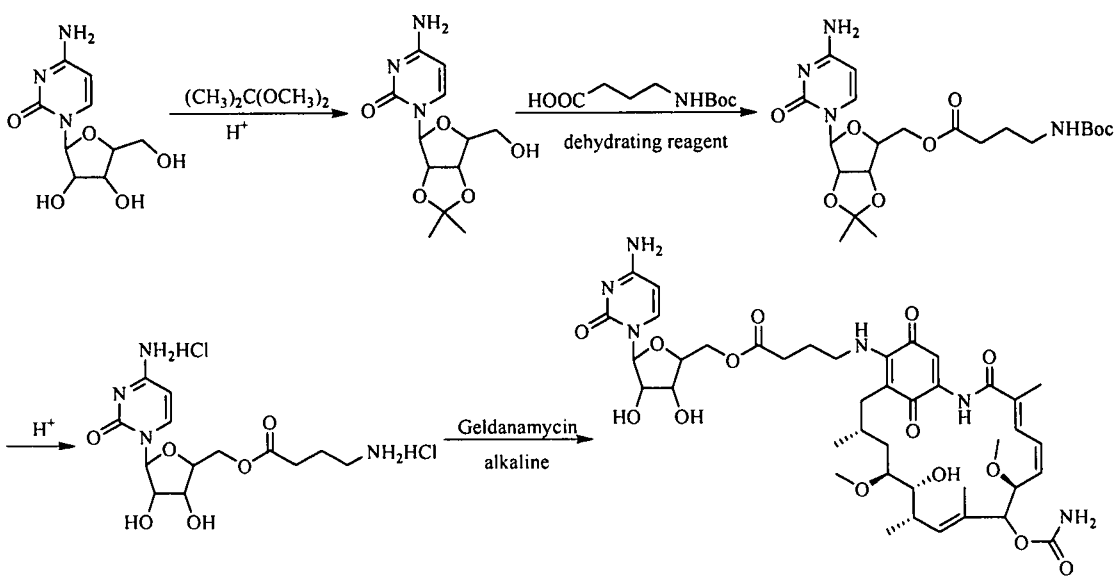


**8.** A method for preparing the geldanamycin derivatives defined in claim 1, wherein , when $R_1$ is a substituent containing a structure of niacinamide structure, the method is as follows, nicotinic acid reacts with acyl chlorinating reagent dichlorosulfoxide to obtain nicotinoyl chloride, which reacts with 2-(N-*tert*-butyloxycarbonyl) ethanediamine to obtain 2-(*tert*-butoxycarbonylamino) ethyl niacinamide, from which the BOC protective group is removed by alcoholysis under acidic catalysis to obtain (2-amino)ethyl nicotinoylamide, which reacts finally with geldanamycin according to the method of claim 2 to obtain geldanamycin derivative containing nicotinoylamide structure linked to the 17-site.

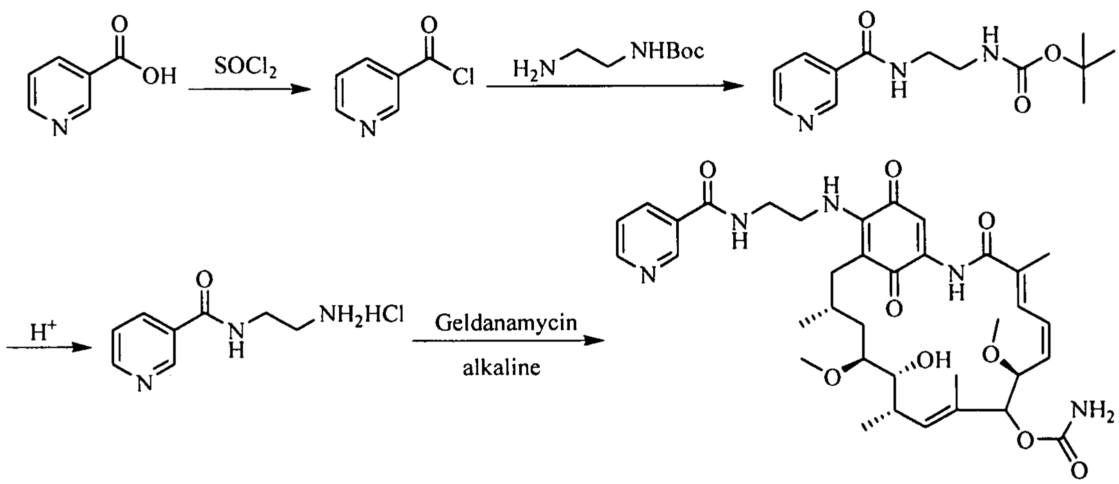


**9.** A method for preparing the geldanamycin derivatives defined in claim 1, wherein, when $R_1$ is a substituent containing a phosphonate group, the method is as follows, *p*-tolyl sulfonyloxoalkyl phosphonate diethyl ester reacts with phthalimide potassium salt in a polar aprotic solvent to produce *N*-alkylphosphonate diethyl ester-phthalimide, which reacts with hydrazine hydrate to produce aminoalkyl phosphonate diethyl ester, which reacts with geldanamycin according to the method of claim 2 to obtain geldanamycin derivative containing a phosphonate group linked to the 17-site.

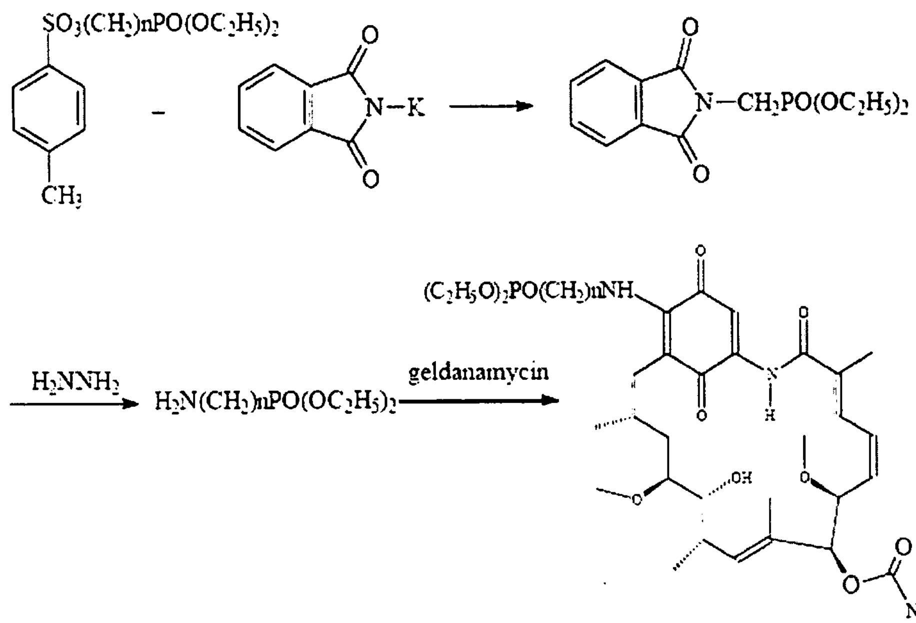


**10.** The pharmaceutical compositions of the compounds shown in Formula (I) of claim 1, wherein said compositions consists of said compounds with therapeutically effective amount as the active components and one or more pharmacologically acceptable carriers.

**11.** The use of the compounds defined in claim 1 for preparing anti-virus and anti-tumor medicines.

**12.** The use of the compositions defined in claim 10 for preparing anti-virus and anti-tumor medicines.

# INTERNATIONAL SEARCH REPORT

International application No.
PCT/CN2009/000074

A. CLASSIFICATION OF SUBJECT MATTER

See extra sheet

According to International Patent Classification (IPC) or to both national classification and IPC

B. FIELDS SEARCHED

Minimum documentation searched (classification system followed by classification symbols)

IPC: C07D A61K A61P

Documentation searched other than minimum documentation to the extent that such documents are included in the fields searched

Electronic data base consulted during the international search (name of data base and, where practicable, search terms used)

WPI, EPODOC, PAJ, CNPAT, CNKI, CA: Geldanamycin 1044760-!!-! 1044759-!!-! 1044758-!!-!

C. DOCUMENTS CONSIDERED TO BE RELEVANT

| Category* | Citation of document, with indication, where appropriate, of the relevant passages | Relevant to claim No. |
|---|---|---|
| PX | CN101302199A(INST MEDICAL BIOTECHNOLOGY CHINESE ACA),12 Nov.2008 (12.11.2008), see claims 1-5 | 1,10-12 |
| PX | CN101220068A(INST MEDICAL BIOLOGY CAMS),16 July 2008(16.07.2008), see the whole document | 1-12 |
| X | US2006205705A1(UNIV COLORADO),14 Sep.2006(14.09.2006), see columns 3-5, compounds in [0036] and [0047] | 1,10-12 |
| X | CN101072504A(CONFORMA THERAPEUTICS CORP),14 Nov.2007(14.11.2007), see compound 237 | 1 |
| X | US2005267046A1(KOSAN BIOSCIENCES INC),01 Dec.2005 (01.12.2005), see compounds of formulae I-VI, especially formula III, claim 1 | 1,10-12 |

☒ Further documents are listed in the continuation of Box C. ☒ See patent family annex.

* Special categories of cited documents:

"A" document defining the general state of the art which is not considered to be of particular relevance

"E" earlier application or patent but published on or after the international filing date

"L" document which may throw doubts on priority claim (S) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&"document member of the same patent family

| Date of the actual completion of the international search | Date of mailing of the international search report |
|---|---|
| 30 March 2009 (30.03.2009) | **30 Apr. 2009 (30.04.2009)** |

| Name and mailing address of the ISA/CN | Authorized officer |
|---|---|
| The State Intellectual Property Office, the P.R.China<br>6 Xitucheng Rd., Jimen Bridge, Haidian District, Beijing, China 100088<br>Facsimile No. 86-10-62019451 | HE Xiaoping<br>Telephone No. (86-10)62084365 |

Form PCT/ISA/210 (second sheet) (April 2007)

## INTERNATIONAL SEARCH REPORT

International application No.
PCT/CN2009/000074

C (Continuation). DOCUMENTS CONSIDERED TO BE RELEVANT

| Category* | Citation of document, with indication, where appropriate, of the relevant passages | Relevant to claim No. |
|---|---|---|
| X | US2005267122A1(CONFORMA THERAPEUTIC CORP), 01 Dec.2005 (01.12.2005), see columns 1-2,14-17,examples 72-74,78-80,82 and compounds 482,709,736,996 | 1,10-12 |
| Y | | 5-6,8-9 |
| X | WO03013430A2(KOSAN BIOSCIENCES INC),20 Feb.2003(20.02.2003), see scheme 2, fig. 2 | 1,10-12 |
| X | CN1817866A(MEDICAL & BIO TECHNOLOGY INST CHINA MEDICAL ACA), 16 Aug. 2006 (16.08.2006), see claims 1-4, pages 4-7, compounds of table 1 of description | 1-4,7,10-12 |
| Y | | 5-6,8-9 |

Form PCT/ISA/210 (continuation of second sheet ) (April 2007)

**INTERNATIONAL SEARCH REPORT**
Information on patent family members

International application No.
PCT/CN2009/000074

| Patent Documents referred in the Report | Publication Date | Patent Family | Publication Date |
|---|---|---|---|
| CN101302199A | 12.11.2008 | none | |
| CN101220068A | 16.07.2008 | none | |
| US2006205705A1 | 14.09.2006 | WO2006098761A2 | 21.09.2006 |
| | | EP1863769A2 | 12.12.2007 |
| CN101072504A | 14.11.2007 | WO2006050457A2 | 11.05.2006 |
| | | EP1814392A2 | 08.08.2007 |
| | | NO20072190A | 13.07.2007 |
| | | AU2005302000A1 | 11.05.2006 |
| | | INDELNP200702855E | 13.07.2007 |
| | | KR20070085677A | 27.08.2007 |
| | | JP2008519031T | 05.06.2008 |
| | | MXPA07004893A | 01.07.2007 |
| US2005267046A1 | 01.12.2005 | WO2005112952A2 | 01.12.2005 |
| | | EP1747005A2 | 31.01.2007 |
| | | US7259156B2 | 21.08.2007 |
| | | JP2008505984T | 28.02.2008 |
| US2005267122A1 | 01.12.2005 | WO03066005A2 | 14.08.2003 |
| | | AU2003217393A1 | 02.09.2003 |
| | | EP1472230A2 | 03.11.2004 |
| | | JP2005530689T | 13.10.2005 |
| WO03013430A2 | 20.02.2003 | EP1420747A2 | 26.05.2004 |
| | | AU2002330998A1 | 24.02.2003 |
| | | JP2005515164T | 26.05.2005 |
| | | US2005261263A1 | 24.11.2005 |
| | | AU2002330998A8 | 27.10.2005 |
| | | US7405208B2 | 29.07.2008 |
| CN1817866A | 16.08.2006 | none | |

Form PCT/ISA/210 (patent family annex) (April 2007)

## INTERNATIONAL SEARCH REPORT

International application No.
PCT/CN2009/000074

CLASSIFICATION OF SUBJECT MATTER

C07D225/06 (2006.01) i
C07H19/06 (2006.01) n
C07D401/12 (2006.01) n
A61K 31/395 (2006.01) n
A61K 31/4427 (2006.01) n
A61P 31/12 (2006.01) n
A61P 35/00 (2006.01) n

Form PCT/ISA/210 (extra sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 1817866 A


### Non-patent literature cited in the description


- **Li Yuhuan ; Tao Pei-Heng et al.** *Antimicrobial Agents and Chemotherapy,* 2004, vol. 48 (3), 867-872
- **Zhao Zhizhong et al.** Protecting Groups in Organic Chemistry. Science Press, 1984, 41-49